# EUROPEAN PATENT APPLICATION

(11) **EP 4 643 761 A1**
(43) Date of publication of application: **05.11.2025**
(21) Application number: 23911920.9
(22) Date of filing: 21.12.2023
(51) Int. Cl.: A61B 3/12, A61B 3/14

(54) **OPHTHALMIC DEVICE, OPHTHALMIC DEVICE CONTROL METHOD, OPHTHALMIC IMAGING METHOD, PROGRAM, AND RECORDING MEDIUM**

(30) Priority: 27.12.2022 JP 2022209392
(71) Applicant: Topcon Corporation, Tokyo 174-8580 (JP)
(72) Inventor: YAMABE Masaru, Tokyo 174-8580 (JP); FUJII Kohta, Tokyo 174-8580 (JP); MATSUI Takuya, Tokyo 174-8580 (JP)
(74) Representative: Gagel, Roland
(86) International application number: PCT/JP2023/045884
(87) International publication number: WO 2024/143140

(57) **Abstract**

An imaging unit of an ophthalmic device according to an embodiment performs imaging with a rolling-shutter-type imaging device while moving a projection position of slit light to the fundus of an eye-to-be-examined. A focus adjustment unit has a configuration for adjusting the focus of the imaging unit. A processor executes a slit light projection control, a condition determination process, and a focus adjustment control. In the slit light projection control, the processor controls the imaging unit for projecting the slit light to the fundus of the eye-to-be-examined. In the condition determination process, the processor determines the focus adjustment condition on the basis of an output from the imaging device that detects return light of the slit light that has been projected to the fundus. In the focus adjustment control, the processor controls the focus adjustment unit on the basis of this focus adjustment condition.

## Description

### [TECHNICAL FIELD]

The present disclosure generally relates to an ophthalmic apparatus, a method of controlling an ophthalmic apparatus, an ophthalmic imaging method, a program, and a recording medium.

### [BACKGROUND OF THE INVENTION]

Examples of known ophthalmic apparatuses configured for imaging (capturing images, photographing) a fundus of a subject's eye, include fundus cameras and scanning laser ophthalmoscopes (SLOs). Patent Document 1 set forth below discloses an ophthalmic apparatus configured to illuminate an eye fundus with slit light and detect return light of the slit light using a rolling shutter type imaging device (CMOS), thereby generating a fundus image. This ophthalmic apparatus is capable of acquiring a fundus image with a simple configuration by repeating the movement of the projection position of the slit light relative to the fundus and the detection of the return light (i.e., the imaging) by the imaging device in synchronization with each other.

### [PRIOR ART DOCUMENTS]

### [PATENT DOCUMENTS]

[PATENT DOCUMENT 1] U.S. Patent No. 7,831,106

### [BRIEF SUMMARY OF THE INVENTION]

### [PROBLEM TO BE SOLVED BY THE INVENTION]

Known ophthalmic apparatuses, such as the ophthalmic apparatus disclosed in Patent Document 1, is provided with a dedicated hardware component for focus adjustment, which may give rise to various concerns including increased apparatus sizes, more complex structures, and higher manufacturing costs.

One object of the present disclosure is to provide a novel technique that enables focus adjustment without the need to incorporate a dedicated hardware component for focusing operations, as is provided in conventional ophthalmic apparatuses of similar types.

### [MEANS FOR SOLVING THE PROBLEM]

An aspect of some embodiment examples is an ophthalmic apparatus includes an imaging unit, a focus adjusting unit, and a processor. The imaging unit is configured to perform imaging with an imaging device while moving a projection position of slit light relative to a fundus of a subject's eye. The focus adjusting unit is configured for performing focus adjustment of the imaging unit. The processor is configured to perform slit light projection control to control the imaging unit to project slit light onto the fundus, a condition determination process to determine a focus adjustment condition based on an output from the imaging device that has detected return light of the slit light projected under the slit light projection control, and focus adjustment control to control the focus adjusting unit based on the focus adjustment condition determined by the condition determination process.

Another aspect of some embodiment examples is a method for controlling an ophthalmic apparatus. The ophthalmic apparatus includes an imaging unit that performs imaging with an imaging device while moving a projection position of slit light relative to a fundus of a subject's eye, a focus adjusting unit for performing focus adjustment of the imaging unit, a processor, and a memory. The method causes the processor to perform a first control step, a condition determination step, and a second control step. In the first control step, the method causes the processor to control the imaging unit to project slit light onto the fundus. In the condition determination step, the method causes the processor to determine a focus adjustment condition based on an output from the imaging device that has detected return light of the slit light projected by the first control step. In the second control step, the method causes the processor to control the focus adjusting unit based on the focus adjustment condition determined by the condition determination step.

Another aspect of some embodiment examples is a method for performing imaging with an imaging device while moving a projection position of slit light relative to a fundus of a subject's eye. The method includes a step of projecting slit light onto the fundus and detecting return light of the slit light projected onto the fundus by the imaging device. Furthermore, the method includes a step of determining a focus adjustment condition based on an output from the imaging device that has detected the return light, and a step of performing focus adjustment based on the focus adjustment condition. In addition, the method includes a step of performing imaging with the imaging device while moving a projection position of slit light relative to the fundus after the focus adjustment has been performed.

Another aspect of some embodiment examples is a program executed by an ophthalmic apparatus. The ophthalmic apparatus includes an imaging unit that performs imaging with an imaging device while moving a projection position of slit light relative to a fundus of a subject's eye, a focus adjusting unit for performing focus adjustment of the imaging unit, a processor, and a memory. The program is configured to cause the processor to perform a first control step, a condition determination step, and a second control step. In the first control step, the program causes the processor to control the imaging unit to project slit light onto the fundus. In the condition determination step, the program causes the processor to determine a focus adjustment condition based on an output from the imaging device that has detected return light of the slit light projected by the first control step. In the second control step, the program causes the processor to control the focus adjusting unit based on the focus adjustment condition determined by the condition determination step.

Another aspect of some embodiment examples is a program for causing a computer. The computer includes a processor and a memory to execute processing for performing imaging with an imaging device while moving a projection position of slit light relative to a fundus of a subject's eye. The program is configured to cause the processor to perform a step of executing control to project slit light onto the fundus and detect return light of the slit light projected onto the fundus by the imaging device. Further, the program is configured to cause the processor to perform a step of determining a focus adjustment condition based on an output from the imaging device that has detected the return light, and a step of performing focus adjustment based on the focus adjustment condition. In addition, the program is configured to cause the processor to perform a step of executing control to perform imaging with the imaging device while moving a projection position of slit light relative to the fundus after the focus adjustment has been performed.

Another aspect of some embodiment examples is a computer-readable non-transitory recording medium storing the program of any aspect of embodiments.

### [EFFECT OF THE INVENTION]

According to some embodiment examples, it becomes possible to perform focus adjustment without having to incorporate a dedicated hardware component for focusing operations, as is provided in conventional ophthalmic apparatuses of similar types.

### [BRIEF DESCRIPTION OF THE SEVERAL VIEWS OF THE DRAWING]

FIG. 1 is a schematic diagram for describing a non-limiting example of the configuration of an ophthalmic apparatus according to an embodiment.
FIG. 2 is a schematic diagram for describing a non-limiting example of the configuration of an ophthalmic apparatus according to an embodiment.
FIG. 3 is a schematic diagram for describing a non-limiting example of the configuration of an ophthalmic apparatus according to an embodiment.
FIG. 4 is a schematic diagram for describing a non-limiting example of the configuration of an ophthalmic apparatus according to an embodiment.
FIG. 5 is a schematic diagram for describing a non-limiting example of the operation of an ophthalmic apparatus according to an embodiment.
FIG. 6 is a schematic diagram for describing a non-limiting example of the configuration of an ophthalmic apparatus according to an embodiment.
FIG. 7 is a schematic diagram for describing a non-limiting example of the optical path formed by an ophthalmic apparatus according to an embodiment.
FIG. 8 is a schematic diagram for describing a non-limiting example of the focus adjustment performed by an ophthalmic apparatus according to an embodiment.
FIG. 9 is a schematic diagram for describing a non-limiting example of the focus adjustment performed by an ophthalmic apparatus according to an embodiment.
FIG. 10 is a schematic diagram for describing a non-limiting example of the focus adjustment performed by an ophthalmic apparatus according to an embodiment.
FIG. 11 is a schematic diagram for describing a non-limiting example of the focus adjustment performed by an ophthalmic apparatus according to an embodiment.
FIG. 12A is a schematic diagram for describing a non-limiting example of the focus adjustment performed by an ophthalmic apparatus according to an embodiment.
FIG. 12B is a schematic diagram for describing a non-limiting example of the focus adjustment performed by an ophthalmic apparatus according to an embodiment.
FIG. 12C is a schematic diagram for describing a non-limiting example of the focus adjustment performed by an ophthalmic apparatus according to an embodiment.
FIG. 12D is a schematic diagram for describing a non-limiting example of the focus adjustment performed by an ophthalmic apparatus according to an embodiment.
FIG. 13A is a schematic diagram for describing a non-limiting example of the focus adjustment performed by an ophthalmic apparatus according to an embodiment.
FIG. 13B is a schematic diagram for describing a non-limiting example of the focus adjustment performed by an ophthalmic apparatus according to an embodiment.
FIG. 14 is a flowchart for describing a non-limiting example of the operation performed by an ophthalmic apparatus according to an embodiment.
FIG. 15 is a schematic diagram for describing a non-limiting example of the operation performed by an ophthalmic apparatus according to an embodiment.
FIG. 16 is a schematic diagram for describing a non-limiting example of the operation performed by an ophthalmic apparatus according to an embodiment.
FIG. 17 is a schematic diagram for describing a non-limiting example of the operation performed by an ophthalmic apparatus according to an embodiment.
FIG. 18 is a flowchart for describing a non-limiting example of the operation performed by an ophthalmic apparatus according to an embodiment.
FIG. 19 is a schematic diagram for describing a non-limiting example of the operation performed by an ophthalmic apparatus according to an embodiment.
FIG. 20 is a flowchart for describing a non-limiting example of the operation performed by an ophthalmic apparatus according to an embodiment.
FIG. 21 is a schematic diagram for describing a non-limiting example of the configuration of an ophthalmic apparatus according to an embodiment.
FIG. 22 is a schematic diagram for describing a non-limiting example of the operation performed by an ophthalmic apparatus according to an embodiment.
FIG. 23A is a schematic diagram for describing a non-limiting example of the operation performed by an ophthalmic apparatus according to an embodiment.
FIG. 23B is a schematic diagram for describing a non-limiting example of the operation performed by an ophthalmic apparatus according to an embodiment.
FIG. 23C is a schematic diagram for describing a non-limiting example of the operation performed by an ophthalmic apparatus according to an embodiment.
FIG. 24 is a schematic diagram for describing a non-limiting example of the configuration of an ophthalmic apparatus according to an embodiment.
FIG. 25 is a schematic diagram for describing a non-limiting example of the configuration of an ophthalmic apparatus according to an embodiment.
FIG. 26 is a schematic diagram for describing a non-limiting example of the configuration of an ophthalmic apparatus according to an embodiment.
FIG. 27 is a schematic diagram for describing a non-limiting example of display information provided by an ophthalmic apparatus according to an embodiment.
FIG. 28 is a schematic diagram for describing a non-limiting example of display information provided by an ophthalmic apparatus according to an embodiment.

### [DETAILED DESCRIPTION OF THE INVENTION]

Several non-limiting aspect examples of embodiment examples according to the present disclosure will be described in detail with reference to the drawings.

Freely selected known techniques or technologies may be combined with any of the aspect examples according to the present disclosure. For example, freely selected matters or items disclosed in the documents cited in the present specification may be combined with any of the aspect examples according to the present disclosure. In addition, freely selected known techniques or technologies in the technical field related to the present disclosure may be combined with any of the aspect examples according to the present disclosure. For instance, freely selected technical matters or items disclosed by the applicant of the present application regarding freely selected techniques or technologies related to the present disclosure or regarding freely selected techniques or technologies applicable to the techniques or technologies related to the present disclosure (e.g., matters or items disclosed in patent applications, academic papers, etc.), may be combined with any of the aspect examples according to the present disclosure.

Freely selected two or more of the various aspect examples described in the present disclosure may be combined, at least in part.

One or more of the functions of the elements (components, parts, units, sections, modules, features, means, constituents, members, devices, mechanisms, or the like) described in the present disclosure can be implemented by using a circuit configuration (circuitry) or a processing circuit configuration (processing circuitry). The circuitry or the processing circuitry includes any of the followings, all of which are configured and/or programmed to execute one or more functions disclosed herein: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), a conventional circuit configuration or circuitry, and any combination of these. A processor is considered to be processing circuitry or circuitry that includes a transistor and/or another circuitry. In the present disclosure, circuitry, a unit, a means, or any terms similar to these is hardware configured to execute one or more functions disclosed herein, or hardware that is programmed to execute one or more functions disclosed herein. The hardware may be any hardware disclosed in the present specification, or alternatively, known hardware that is programmed and/or configured to execute one or more functions described herein. In the case where the hardware is a processor, which may be considered as a certain type of circuitry, then circuitry, a unit, a means, or any terms similar to these is a combination of hardware and software. In this case, the software is used to configure the hardware and/or the processor.

An ophthalmic apparatus according to some embodiment examples includes an imaging unit, a focus adjusting unit, and a processor. The imaging unit is configured to perform imaging of a fundus of a subject's eye with an imaging device while moving a projection position of slit light relative to the fundus of the subject's eye. The imaging device may be an imaging device of a rolling shutter type or another imaging device having similar functionality. As an example of the latter, the imaging device may be one that combines a global shutter type image sensor with a mechanical slit diaphragm. The same may apply to the imaging devices according to other embodiment examples. The imaging technique performed by the ophthalmic apparatus according to the present disclosure may be referred to as slit scanning. The focus adjusting unit is configured to perform focus adjustment of the imaging unit. The processor is configured to perform slit light projection control, a condition determination process, and focus adjustment control. In the slit light projection control, the processor controls the imaging unit so as to project slit light onto the fundus of the subject's eye. In the condition determination process, the processor determines a focus adjustment condition based on an output from the imaging device that has detected return light of the slit light projected onto the fundus of the subject's eye under the slit light projection control. In the focus adjustment control, the processor controls the focus adjusting unit based on the focus adjustment condition determined by the condition determination process.

In the ophthalmic apparatus according to some embodiment examples, the processor may include a condition determining processor configured to perform the condition determination process. The condition determining processor may be configured to determine the focus adjustment condition based on a position of a slit light image in an image generated by the imaging device of the imaging unit.

In some aspect examples of such embodiment examples, the condition determining processor may be configured to determine the position of the slit light image in the image generated by the imaging device of the imaging unit based on an intensity profile in an image region that is at least a part of the slit light image. The intensity profile is determined along a first direction corresponding to a direction of the movement of the projection position of the slit light performed by the imaging unit.

Furthermore, in some aspect examples, the condition determining processor may be configured to generate the intensity profile by summing, in a second direction perpendicular to the first direction, intensities of a group of pixels constituting the image region of the slit light image.

In the ophthalmic apparatus according to some embodiment examples, the imaging unit may include a first optical system and a second optical system. The first optical system is configured to project the slit light onto the fundus of the subject's eye. The second optical system is configured to guide return light of the slit light from the fundus of the subject's eye, to the imaging device. In this case, the focus adjusting unit may include a first focus adjusting unit configured for adjusting a focal position of the first optical system and a second focus adjusting unit configured for adjusting a focal position of the second optical system. In addition, the condition determining processor may be configured to determine, as the focus adjustment condition, a first focus adjustment condition used for controlling the first focus adjusting unit and a second focus adjustment condition used for controlling the second focus adjusting unit.

In some aspect examples of such embodiment examples, the first optical system may include a light source and a slit diaphragm configured for generating slit light from light emitted by the light source, and the first optical system may be configured to project the slit light generated by the slit diaphragm onto the fundus of the subject's eye. Additionally, the second optical system may include a focus lens. Furthermore, the first focus adjusting unit may include a first moving mechanism configured for moving the slit diaphragm in a direction along an optical axis of the first optical system, and the second focus adjusting unit may include a second moving mechanism configured for moving the focus lens in a direction along an optical axis of the second optical system. Moreover, the condition determining processor may be configured to determine a first movement control condition used for controlling the first moving mechanism as the first focus adjustment condition, and to determine a second movement control condition used for controlling the second moving mechanism as the second focus adjustment condition.

In addition, in some aspect examples, the first movement control condition may include information indicating a movement direction and a movement distance of the slit diaphragm. Further, the second movement control condition may include information indicating a movement direction and a movement distance of the focus lens.

In some other aspect examples of the embodiment examples in which the imaging unit includes the first optical system and the second optical system, the focus adjusting unit includes the first focus adjusting unit and the second focus adjusting unit, and the condition determining processor is configured to determine the first focus adjustment condition and the second focus adjustment condition, the following configuration may be adapted. The first optical system may include a light source, an iris diaphragm, a lens, and a slit diaphragm. The iris diaphragm is disposed at a position substantially optically conjugate with an iris of the subject's eye and has an aperture through which light emitted by the light source passes. The lens is configured to refract the light that has passed through the aperture of the iris diaphragm. The slit diaphragm is configured for generating slit light from the light refracted by the lens. The first optical system may be configured to project the slit light generated by the slit diaphragm onto the fundus. The second optical system may include a focus lens. Furthermore, the first focus adjusting unit may include a third moving mechanism configured for integrally moving the light source, the iris diaphragm, the lens, and the slit diaphragm in a direction along an optical axis of the first optical system. Additionally, the second focus adjusting unit may include a fourth moving mechanism configured for moving the focus lens in a direction along an optical axis of the second optical system. Moreover, the condition determining processor may be configured to determine a third movement control condition used for controlling the third moving mechanism as the first focus adjustment condition, and to determine a fourth movement control condition used for controlling the fourth moving mechanism as the second focus adjustment condition.

In addition, in some aspect examples, the third movement control condition may include information indicating a movement direction and a movement distance for the integral movement of the light source, the iris diaphragm, the lens, and the slit diaphragm. Also, the fourth movement control condition may include information indicating a movement direction and a movement distance for the movement of the focus lens.

In the ophthalmic apparatus according to some embodiment examples, the processor may be configured to control the imaging unit, in the slit light projection control, to emit the slit light projected onto the fundus of the subject's eye from a position spaced a predetermined distance from an optical axis of the imaging unit. In this case, the condition determining processor may be configured to determine the focus adjustment condition based on the predetermined distance, the position of the slit light image in an image generated by the imaging device of the imaging unit, and a focal length of the imaging unit.

In the ophthalmic apparatus according to some embodiment examples, the imaging unit may include an optical scanner configured to move the projection position of the slit light relative to the fundus of the subject's eye by deflecting the slit light guided to the subject's eye. In this case, the processor may include a projection controller configured to perform the slit light projection control, and the projection controller may be configured to control the imaging unit to project slit light onto the fundus in a state where a deflection direction of the slit light by the optical scanner is fixed. Furthermore, the condition determining processor may be configured to determine the focus adjustment condition based on a position of a slit light image corresponding to the slit light projected onto the fundus of the subject's eye in the state where the deflection direction of the slit light by the optical scanner is fixed.

In the ophthalmic apparatus according to some embodiment examples, the processor may include a projection controller configured to perform the slit light projection control. The projection controller is configured to control the imaging unit to output a first slit light and a second slit light. Here, the projection position of the first slit light and the projection position of the second slit light on the fundus of the subject's eye are different from each other. In this case, the condition determining processor may be configured to determine the focus adjustment condition based on a relative position between a first slit light image corresponding to the first slit light and a second slit light image corresponding to the second slit light.

In the ophthalmic apparatus according to some embodiment examples, the imaging unit may be configured to acquire a time-series image of a fundus of a subject's eye by a combination of repetitive execution of a series of imaging of the fundus of the subject's eye using the imaging device and repetitive execution of a series of movement of the projection position of the slit light relative to the fundus of the subject's eye. In this case, the processor may be configured to perform first imaging control, second imaging control, and a focus information generation process in addition to the slit light projection control, the condition determination process, and the focus adjustment control. In the first imaging control, the processor performs control of the imaging unit to acquire a time-series image of the fundus of the subject's eye. In the second imaging control, the processor performs control of the imaging unit to perform imaging for detecting a focus state of the imaging unit relative to the fundus of the subject's eye. In the focus information generation process, the processor generates focus information indicating the focus state of the imaging unit relative to the fundus of the subject's eye, based on an image acquired with the imaging unit by the imaging performed under the second imaging control. In some aspect examples of such embodiment examples, the processor may be configured to perform the first imaging control and the second imaging control in alternation. Furthermore, in some aspect examples, the processor may further be configured to perform display control to output display information based on the focus information to a display device, in parallel with the first imaging control, the second imaging control, and the focus information generation process.

The ophthalmic apparatus according to some embodiment examples includes an imaging unit and a processor. The imaging unit is capable of acquiring a time-series image of a fundus of a subject's eye by repeatedly performing a series of imaging of the fundus of the subject's eye using the imaging device while repeating a series of movement of the projection position of the slit light relative to the fundus of the subject's eye. The processor may be configured to perform first imaging control, second imaging control, a focus information generation process, and display control. In the first imaging control, the processor performs control of the imaging unit to acquire a time-series image of the fundus of the subject's eye. In the second imaging control, the processor performs control of the imaging unit to conduct imaging for detecting a focus state of the imaging unit relative to the fundus of the subject's eye. In the focus information generation process, the processor generates focus information indicating the focus state of the imaging unit relative to the fundus of the subject's eye, based on an image acquired by the imaging unit by the imaging performed under the second imaging control. In the display control, the processor performs display control to output display information based on the focus information generated by the focus information generation process, to a display device.

A method according to some embodiment examples is a method of controlling an ophthalmic apparatus. The ophthalmic apparatus includes an imaging unit, a focus adjusting unit, a processor, and a memory. The imaging unit is configured to perform imaging of a fundus of a subject's eye with an imaging device while moving a projection position of slit light relative to the fundus of the subject's eye. The focus adjusting unit is configured to perform focus adjustment of the imaging unit. The method according to the present embodiment examples is configured to cause the processor to perform the following steps: a first control step of controlling the imaging unit to project slit light onto the fundus of the subject's eye; a condition determination step of determining a focus adjustment condition based on an output from the imaging device that has detected return light of the slit light projected onto the fundus of the subject's eye by the first control step; and a second control step of controlling the focus adjusting unit based on the focus adjustment condition determined by the condition determination step.

A method according to some embodiment examples is a method of controlling an ophthalmic apparatus. The ophthalmic apparatus includes an imaging unit, a processor, and a memory. The imaging unit is configured to perform imaging of a fundus of a subject's eye with an imaging device while moving a projection position of slit light relative to the fundus of the subject's eye. The method according to the present embodiment examples is configured to cause the processor to perform the following steps in parallel: a first imaging control step of controlling the imaging unit to acquire a time-series image of a fundus of a subject's eye by repeatedly performing a series of imaging of the fundus of the subject's eye using the imaging device while repeating a series of movement of the projection position of the slit light relative to the fundus; a second imaging control step of controlling the imaging unit to perform imaging for detecting a focus state of the imaging unit relative to the fundus of the subject's eye; a focus information generation step of generating focus information indicating the focus state of the imaging unit relative to the fundus of the subject's eye, based on an image acquired by the imaging performed by the second imaging control step; and a display control step of outputting, to a display device, display information based on the focus information generated by the focus information generation step.

A method according to some embodiment examples is a method of performing imaging of a fundus of a subject's eye with an imaging device while moving a projection position of slit light relative to the fundus of the subject's eye. The method according to the present embodiment examples is configured to perform the following steps: a step of projecting slit light onto the fundus of the subject's eye and detecting return light of the slit light projected onto the fundus by the imaging device; a step of determining a focus adjustment condition based on an output from the imaging device that has detected the return light of the slit light from the fundus of the subject's eye; a step of performing focus adjustment based on the focus adjustment condition; and a step of performing imaging of the fundus of the subject's eye with the imaging device while moving a projection position of slit light relative to the fundus of the subject's eye after the focus adjustment has been performed.

A method according to some embodiment examples is a method for performing imaging of a fundus of a subject's eye with an imaging device while moving a projection position of slit light relative to the fundus of the subject's eye. The method according to the present embodiment examples is configured to perform the following steps in parallel: a step of acquiring a time-series image of the fundus of the subject's eye by repeatedly performing a series of imaging of the fundus of the subject's eye using the imaging device while repeating a series of movement of the projection position of the slit light relative to the fundus of the subject's eye; a step of acquiring an image by performing imaging for detecting a focus state relative to the fundus of the subject's eye; a step of generating focus information indicating the focus state based on an image acquired by the imaging for detecting the focus state; and a step of outputting display information based on the focus information.

A program according to some embodiment examples is a program executed by an ophthalmic apparatus. The ophthalmic apparatus includes an imaging unit that performs imaging of a fundus of a subject's eye with an imaging device while moving a projection position of slit light relative to the fundus of the subject's eye, a focus adjusting unit for performing focus adjustment of the imaging unit, a processor, and a memory. The program according to the present embodiment examples is configured to cause the processor to perform the following steps: a first control step of controlling the imaging unit to project slit light onto the fundus of the subject's eye; a condition determination step of determining a focus adjustment condition based on an output from the imaging device that has detected return light of the slit light projected onto the fundus of the subject's eye by the first control step; and a second control step of controlling the focus adjusting unit based on the focus adjustment condition determined by the condition determination step. A recording medium according to some embodiment examples is a computer-readable non-transitory recording medium storing the program according to the present embodiment examples.

A program according to some embodiment examples is a program executed by an ophthalmic apparatus. The ophthalmic apparatus includes an imaging unit that performs imaging of a fundus of a subject's eye with an imaging device while moving a projection position of slit light relative to the fundus of the subject's eye, a processor, and a memory. The program according to the present embodiment examples is configured to cause the processor to perform the following steps in parallel: a first imaging control step of controlling the imaging unit to acquire a time-series image of the fundus of the subject's eye by repeatedly performing a series of imaging of the fundus of the subject's eye using the imaging device while repeating a series of movement of the projection position of the slit light relative to the fundus of the subject's eye; a second imaging control step of controlling the imaging unit to perform imaging for detecting a focus state of the imaging unit relative to the fundus of the subject's eye; a focus information generation step of generating focus information indicating the focus state based on an image acquired by the imaging performed by the second imaging control step; and a display control step of outputting, to a display device, display information based on the focus information generated by the focus information generation step. A recording medium according to some embodiment examples is a computer-readable non-transitory recording medium storing the program according to the present embodiment examples.

A program according to some embodiment examples causes a computer including a processor and a memory, to execute processing for performing imaging of a fundus of a subject's eye with an imaging device while moving a projection position of slit light relative to the fundus of the subject's eye. The program according to the present embodiment examples is configured to cause the processor to perform the following steps: a step of executing control to project slit light onto the fundus of the subject's eye and detect return light of the slit light projected onto the fundus of the subject's eye by the imaging device; a step of determining a focus adjustment condition based on an output from the imaging device that has detected the return light of the slit light projected onto the fundus of the subject's eye; a step of performing focus adjustment based on the focus adjustment condition; and a step of executing control to perform imaging of the fundus of the subject's eye with the imaging device while moving a projection position of slit light relative to the fundus of the subject's eye after the focus adjustment has been performed. A recording medium according to some embodiment examples is a computer-readable non-transitory recording medium storing the program according to the present embodiment examples.

A program according to some embodiment examples causes a computer including a processor and a memory to execute processing for performing imaging of a fundus of a subject's eye with an imaging device while moving a projection position of slit light relative to the fundus of the subject's eye. The program according to the present embodiment examples is configured to cause the processor to perform the following steps in parallel: a step of acquiring a time-series image of the fundus of the subject's eye by repeatedly performing a series of imaging of the fundus of the subject's eye using the imaging device while repeating a series of movement of the projection position of the slit light relative to the fundus of the subject's eye; a step of acquiring an image by performing imaging for detecting a focus state relative to the fundus of the subject's eye; a step of generating focus information indicating the focus state based on an image acquired by the imaging for detecting the focus state; and a step of outputting display information based on the focus information. A recording medium according to some embodiment examples is a computer-readable non-transitory recording medium storing the program according to the present embodiment examples.

### < First embodiment example>

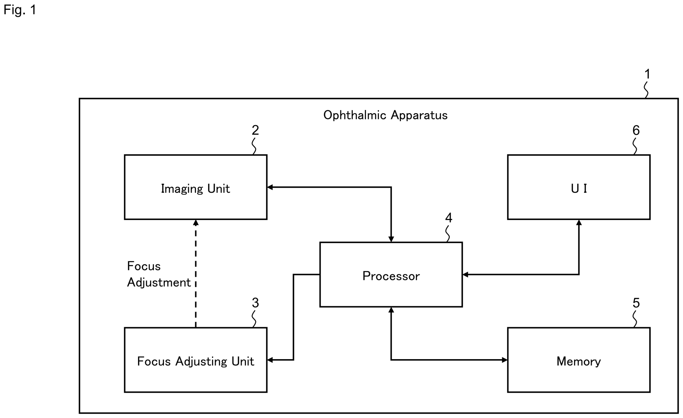

A non-limiting example of the configuration of the ophthalmic apparatus according to the first embodiment example is shown in FIG. 1. The ophthalmic apparatus 1 of the present example has an ophthalmic imaging function that performs imaging (visualization) of the fundus of a living eye using a slit-scanning modality. The ophthalmic apparatus 1 includes the imaging unit 2, the focus adjusting unit 3, the processor 4, the memory 5, and the user interface 6. Unless otherwise specified, the hardware components included in the ophthalmic apparatus 1 may be the same as those of existing slit-scanning type ophthalmic imaging apparatuses.

The imaging unit 2 is configured to be able to conduct slit scanning by performing imaging of the fundus of the subject's eye with an image sensor (imaging device) of a rolling shutter type while moving the projection position of slit light relative to the fundus of the subject's eye. In other words, the imaging unit 2 is configured to illuminate the fundus of the subject's eye with slit-shaped illumination light (slit light) while moving the illumination position (illumination area, illumination region, or the like) of the slit light, and to receive return light from the fundus using an image sensor in which photodetectors are arranged in a one-dimensional or two-dimensional array. Under the control of the processor 4, the result of the reception of the return light is read out as signals (data) from the photodetectors located at the reception positions of the return light corresponding to the illumination area of the slit light, in synchronization with the movement timing of the illumination area of the slit light. The signal readout from the image sensor is performed using the rolling shutter method. Several non-limiting examples of the specific configuration of the imaging unit 2 will be described later.

As previously mentioned, an imaging unit (imaging device) that is a combination an image sensor of a global shutter type and a slit diaphragm can be employed, in place of a rolling shutter type image sensor, in order to conduct imaging operations same as those of rolling shutter type image sensors.

The focus adjusting unit 3 includes a configuration for performing focus adjustment (focusing or focusing operation) of the imaging unit 2. The focus adjustment of the first embodiment example may be performed by the use of an existing focus adjustment technique. For example, focus adjustment may be performed by changing the focal length (the position of the focal point) of the lens disposed between the subject (the fundus of the subject's eye) and the image sensor, and/or by changing the distance between the lens and the image sensor.

The processor 4 can realize the function according to the first embodiment example by executing processing in accordance with a program stored in the memory 5 and/or another storage device.

The memory 5 stores various types of computer programs and various types of data. For example, the memory 5 stores a control program and/or control data configured for causing the ophthalmic apparatus 1 to execute a predetermined operation, and an arithmetic program and/or arithmetic data configured for causing the ophthalmic apparatus 1 to execute predetermined arithmetic processing. The types of programs and data stored in the memory 5 are not limited to the examples described herein. In addition, the memory 5 stores data acquired by the ophthalmic apparatus 1. For example, data generated by the ophthalmic apparatus 1 or data acquired from an external source by the ophthalmic apparatus 1 may be stored in the memory 5. In some typical embodiment examples, the memory 5 includes a non-volatile memory and a volatile memory.

The user interface 6 is a component (hardware component, software component, protocol) configured for exchanging information between the ophthalmic apparatus 1 and its user. The user interface 6 includes, for example, an input unit (operation unit) that serves as a means for providing information from the user to the ophthalmic apparatus 1, and an output unit that serves as a means for providing information from the ophthalmic apparatus 1 to the user. Non-limiting examples of the hardware component of the input unit include an operation panel, a mouse, a keyboard, a trackball, a switch, a button, a dial, a scanner, an optical character recognition (OCR) device, a microphone, and a camera (video camera). Non-limiting examples of the hardware component of the output unit include a display device, a printer, and a speaker. The user interface 6 may include a device in which an input function and an output function are integrated into a single unit, such as a touch screen.

Although not illustrated in the drawings, the ophthalmic apparatus 1 is provided with a component configured for performing alignment of the imaging unit 2 relative to the subject's eye E, similar to existing ophthalmic apparatuses of similar types. The alignment method employed by the ophthalmic apparatus 1 may be freely selected or determined, and may be any of the following methods, for example: stereo alignment of determining the position of the subject's eye E by the use of two or more anterior segment cameras as described in Japanese Unexamined Patent Application Publication No. 2013-248376; a method of determining the position of the subject's eye E by analyzing a front image of the subject's eye E (e.g., an observation image of the anterior segment Ea); and a method of determining the position of the subject's eye E by projecting an alignment indicator onto the anterior segment Ea (cornea). The ophthalmic apparatus 1 includes a hardware component and a software component in accordance with a selected alignment method. Although not shown in the drawings, the ophthalmic apparatus 1 includes a moving mechanism for moving the imaging unit 2 in a three-dimensional manner, similar to existing ophthalmic apparatuses of similar types.

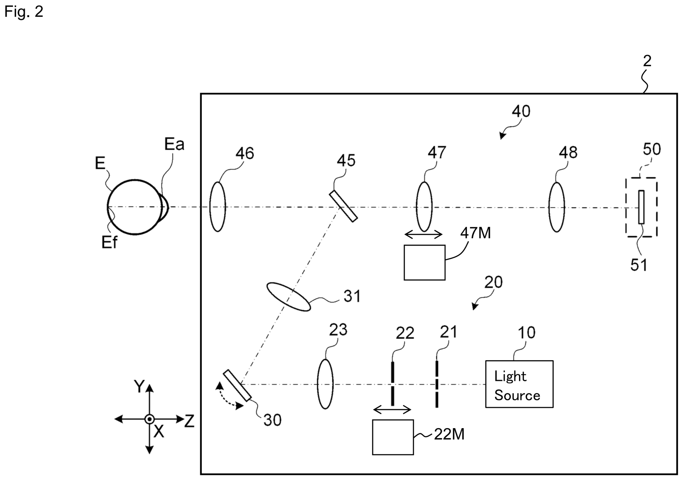

A non-limiting example of the configuration of the imaging unit 2 is shown in FIG. 2. FIG. 2 is a side view. In FIG. 2, the direction along the optical axis of the optical system (the optical axis of the objective lens 46) is defined as the Z direction (front-rear direction, working distance direction). One of the directions perpendicular to the Z direction is defined as the X direction (left-right direction, horizontal direction in the present example), and the direction perpendicular to both the Z direction and the X direction is defined as the Y direction (up-down direction, vertical direction in the present example).

The imaging unit 2 in the present example includes the light source 10, the illumination optical system 20, the optical scanner 30, the photographing optical system 40, and the imaging device 50. The illumination optical system 20 generates slit light from light emitted from the light source 10 and projects the slit light onto the fundus Ef of the subject's eye E. The light source 10 may be considered as a component of the illumination optical system 20. The optical scanner 30 moves the position (projection position) of the slit light projected onto the fundus Ef by the illumination optical system 20. The optical scanner 30 may be considered as a component of the illumination optical system 20. The photographing optical system 40 guides return light of the slit light projected onto the fundus Ef by the illumination optical system 20 to the imaging device 50. The imaging device 50 may be regarded as a component of the photographing optical system 40.

The light source 10 may include a visible light source that emits light within the visible spectral range. An example of the visible light source is a white light source that emits white light. The light source 10 may also include an infrared light source (near-infrared light source) that emits light within the infrared spectral range (near-infrared spectral range). The light source 10 may be capable of switching emitted light between light of different wavelength bands. The light source 10 may include any type of light source, and may include, for example, one or more of the following: a light-emitting diode (LED), a laser diode (LD), a halogen lamp, and a xenon lamp. In a state where the imaging unit 2 is appropriately aligned with the subject's eye E, the light source 10 is disposed at a position optically non-conjugate with both the fundus Ef and the iris. In some aspect examples, under the control of the processor 4, the light source 10 outputs visible light for slit scanning and outputs near-infrared light or visible light for focus adjustment.

The illumination optical system 20 generates slit-shaped illumination light (slit light) from the light emitted by the light source 10, and projects the slit light onto the fundus Ef of the subject's eye E. In the present example, the illumination optical system 20 includes the iris diaphragm 21, the slit aperture diaphragm 22 (the slit 22), the relay lens 23, the optical scanner 30, the relay lens 31, the hole mirror 45, and the objective lens 46. The relay lens 23 includes one or more lenses, the relay lens 31 includes one or more lenses, and the objective lens 46 includes one or more lenses.

The photographing optical system 40 guides return light of the illumination light (light of the slit light) projected onto the fundus Ef of the subject's eye E by the illumination optical system 20 (and the optical scanner 30), to the imaging device 50. In the present example, the photographing optical system 40 includes the objective lens 46, the hole mirror 45, the focus lens 47, and the imaging lens 48. The focus lens 47 includes one or more lenses, and the imaging lens 48 includes one or more lenses.

The light output from the light source 10 (specifically, a part of this light) passes through an aperture formed in the iris diaphragm 21, an aperture formed in the slit aperture diaphragm 22, and the relay lens 23, and is guided to the optical scanner 30.

In a state where the alignment of the imaging unit 2 relative to the subject's eye E is appropriately established, the iris diaphragm 21 (specifically, the aperture formed in the iris diaphragm 21) is disposed at a position that is substantially optically conjugate with the iris (pupil) of the subject's eye E.

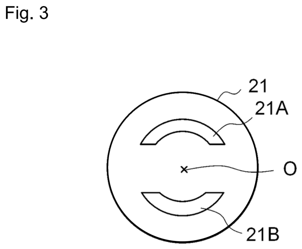

The iris diaphragm 21 has one or more apertures formed at a position(s) spaced from the optical axis of the illumination optical system 20. For example, as illustrated in FIG. 3, a non-limiting example of the iris diaphragm 21 is formed with two apertures 21A and 21B, each having predetermined dimensions (predetermined length and predetermined width) formed curved along the circumferential direction centered on the optical axis O of the illumination optical system 20.

The aperture(s) of the iris diaphragm 21 defines the incident state (incident position, incident shape) of the illumination light at the pupil of the subject's eye E. For instance, when the iris diaphragm 21 with the apertures 21A and 21B is employed, and the optical axis O of the illumination optical system 20 (i.e., the optical axis of the objective lens 46) is positioned so as to substantially coincide with the pupil center of the subject's eye E (i.e., the state of appropriate alignment), the illumination light (slit light) can be guided to the fundus Ef through regions that are shifted from the pupil center (specifically, through two regions arranged in point symmetry with respect to the pupil center.

In some aspect examples, an optical element that deflects light emitted by the light source 10 may be provided between the light source 10 and the iris diaphragm 21 in order to optimize the light intensity distribution in the direction connecting the aperture of the iris diaphragm 21 and the aperture (the slit) of the slit aperture diaphragm 22. Furthermore, the light intensity distribution of the light passing through the aperture of the iris diaphragm 21 may be varied by employing a configuration that allows the relative position between the light source 10 and the aperture of the iris diaphragm 21 to be adjustable.

In a state where the alignment of the imaging unit 2 relative to the subject's eye E is appropriately established, the slit aperture diaphragm 22 (specifically, the aperture (the slit) formed in the slit aperture diaphragm 22) is disposed at a position that is substantially optically conjugate with the fundus Ef of the subject's eye E.

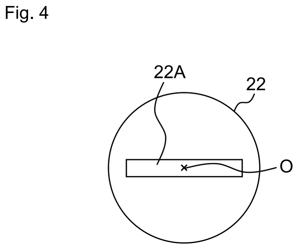

The slit aperture diaphragm 22 has an aperture (slit) formed with its longitudinal direction corresponding to the line direction (row direction) in which signals are read out from the image sensor 51 (described later) using the rolling shutter method. For example, as illustrated in FIG. 4, a non-limiting example of the slit aperture diaphragm 22 has the aperture (the slit) 22A, the area of which has predetermined dimensions (predetermined length and predetermined width) and the optical axis O of the illumination optical system 20 passes through the area.

The aperture (the slit) formed in the slit aperture diaphragm 22 defines the shape of the projected image of the slit light on the fundus Ef of the subject's eye E. The longitudinal direction of the slit formed in the slit aperture diaphragm 22 may be referred to as the slit length direction. Also, the lateral direction (short direction, transverse direction) of the slit formed in the slit aperture diaphragm 22 may be referred to as the slit width direction.

The slit aperture diaphragm 22 is movable in a direction along the optical axis of the illumination optical system 20 by means of the moving mechanism 22M. The moving mechanism 22M operates under the control of the processor 4. The processor 4 may be configured to control the moving mechanism 22M depending on the condition of the subject's eye E. The condition of the subject's eye E may include, for example, refractive power (refractive index, diopter), fundus shape, or other parameters.

The light that has passed through the aperture of the iris diaphragm 21 is converted into slit-shaped illumination light (slit light) by passing through the aperture of the slit aperture diaphragm 22. The slit light is guided to the optical scanner 30 via the relay lens 23, deflected by the optical scanner 30, and then guided to the hole mirror 45 via the relay lens 31.

The hole mirror 45 is an optical member used in conventional fundus cameras and the like apparatuses, and the hole mirror 45 functions as an optical path coupling member that couples the optical path of the illumination optical system 20 and the optical path of the photographing optical system 40 with each other. An aperture (or a light-transmitting part) is formed at the central position of the hole mirror 45. In some examples, the outer edge of this aperture is of a circular shape. The optical axis of the illumination optical system 20 and the optical axis of the photographing optical system 40 intersect with each other at the aperture of the hole mirror 45. A reflecting part (a mirror part) is formed around the aperture of the hole mirror 45.

The slit light guided to the hole mirror 45 via the relay lens 31 is then reflected by the reflecting part and refracted by the objective lens 46, entering the subject's eye E. The slit light that has entered the subject's eye E is projected onto the fundus Ef.

In some aspect examples, the projected image (projection region) of the slit light on the fundus Ef has a substantially slit-like shape, and the longitudinal direction of this slit-shaped projected image substantially coincides with the X direction. In this case, the optical scanner 30 deflects the slit light generated by the iris diaphragm 21 and the slit aperture diaphragm 22 so as to move the projected image of the slit light on the fundus Ef in the Y direction. It should be noted that the orientation of the longitudinal direction of the projected image of the slit light on the fundus Ef is not limited to the X direction, and that the direction in which the optical scanner 30 moves the projected image is not limited to the Y direction.

In a state where the alignment of the imaging unit 2 relative to the subject's eye E is appropriately established, the optical scanner 30 is disposed at a position that is substantially optically conjugate with the iris of the subject's eye E. This arrangement allows the slit light to be deflected in the Y direction, with a position in the pupil of the subject's eye E (or a position near the pupil) serving as a scan pivot (i.e., a scan axis, a deflection center axis). Consequently, it becomes possible to sequentially project the slit light onto a plurality of slit-shaped partial regions formed by virtually dividing a predetermined scan area of the fundus Ef. In other words, it becomes possible to apply slit scanning to the fundus Ef.

The reflected light of the slit light projected onto the fundus Ef exits the subject's eye E through the pupil of the subject's eye E and enters the imaging unit 2. The light (return light) that has entered the imaging unit 2 from the subject's eye E is guided to the hole mirror 45 via the objective lens 46. The return light passes through the aperture of the hole mirror 45 (or passes through the light-transmitting part of the hole mirror 45), and is then guided to the imaging device 50 via the focus lens 47 and the imaging lens 48, where the return light is detected.

The optical scanner 30 may, for example, is operated to deflect the slit light in a one-dimensional or two-dimensional manner. The optical scanner 30 for one-dimensional deflection is operated to deflect the slit light within a predetermined deflection angle range defined with reference to a predetermined reference direction. This deflection angle range is defined in a direction corresponding to the movement direction (for example, the Y direction) of the slit light on the fundus Ef. The optical scanner 30 for two-dimensional deflection is, for example, a combination of two optical scanners, each providing a deflection direction different from that of the other. The type of optical deflection device used in the optical scanner 30 may be freely selected or determined. In some examples, the optical deflection device may be a galvanometer scanner.

The focus lens 47 may be operated to move in a direction along the optical axis of the photographing optical system 40 by the moving mechanism 47M. The moving mechanism 47M operates under the control of the processor 4. The processor 4 may be configured to control the moving mechanism 47M depending on the condition of the subject's eye E (e.g., refractive power, fundus shape, etc.).

The imaging device 50 includes the image sensor 51 that detects the return light guided by the photographing optical system 40. Under the control of the processor 4, the imaging device 50 may perform signal readout from the image sensor 51 in response to detection of the return light.

The image sensor 51 implements the function of a pixelated photodetector. In a state where the alignment of the imaging unit 2 relative to the subject's eye E is appropriately established, the light-receiving surface (also referred to as the detection surface, the imaging surface, the image capturing surface, the light-receiving surface, or the like) of the image sensor 51 is disposed at a position that is substantially optically conjugate with the fundus Ef of the subject's eye E. The signal generated by the image sensor 51 through photoelectric conversion is read out by means of the rolling shutter method under the control of the processor 4.

In some aspect examples, the image sensor 51 includes a CMOS image sensor. In this case, the image sensor 51 is provided with a plurality of pixel groups, each of which is disposed along the row direction, and the plurality of pixel groups is arranged along a column direction. More specifically, the image sensor 51 includes a plurality of pixels arranged as a two-dimensional array, a plurality of vertical signal lines, and a horizontal signal line. Each pixel includes a photodiode and a capacitor. The vertical signal lines are respectively provided for corresponding pixel groups, each of which includes pixels arranged in the column direction (vertical direction) perpendicular to the row direction (horizontal direction). Each vertical signal line is selectively and electrically connected to a corresponding pixel group in which electric charge corresponding to the detection result of the return light is accumulated. The horizontal signal line is selectively and electrically connected to the plurality of vertical signal lines. Each pixel accumulates electric charge corresponding to the detection result of the return light, and the accumulated electric charge is sequentially read out, for example, for each pixel group in the row direction. For instance, for each line in the row direction, voltage corresponding to the electric charge accumulated in each pixel is supplied to the vertical signal line. The plurality of vertical signal lines is selectively and electrically connected to the horizontal signal line. By performing the above-mentioned sequential readout operation for the lines in the row direction in the vertical direction, the detection results can be read out from the plurality of pixels arranged in a two-dimensional array.

By performing such a readout operation of the detection result from the image sensor 51 using the rolling shutter method, it becomes possible to acquire a received image corresponding to a desired virtual aperture shape extending in the row direction. Such control technique is known and is disclosed, for example, in the specification of U.S. Patent No. 7,831,106.

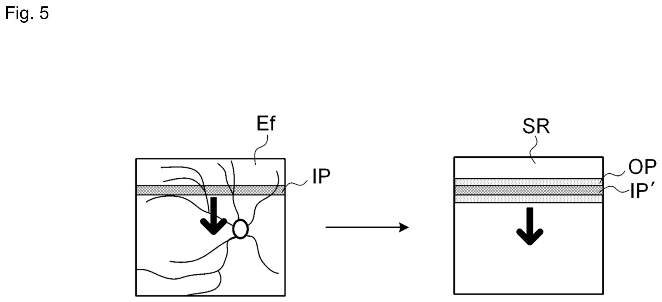

The slit scanning performed by the ophthalmic apparatus 1 will now be described. FIG. 5 shows a schematic diagram of the position (projection area) IP of the projected image of the slit light on the fundus Ef and the virtual aperture area OP on the light-receiving surface SR of the image sensor 51.

The ophthalmic apparatus 1 deflects the slit light using the optical scanner 30, thereby moving the projection area IP of the slit light on the fundus Ef in a direction perpendicular to the slit length direction. Here, the slit length direction is, for example, the X direction, the row direction, or the horizontal direction, and the direction perpendicular to the slit length direction is, for example, the Y direction, the column direction, or the vertical direction.

During signal readout from the image sensor 51 by the processor 4, the virtual aperture area OP is sequentially determined by sequentially switching a pixel group to be read out on a line-by-line basis. The aperture area OP is determined, for example, so as to match the area IP' of the light-receiving surface SR onto which the return light of the slit light is projected, or so as to be an area wider than the area IP'. The processor 4 performs control to move the projection area IP of the slit light and control to move the aperture area OP in parallel. For example, the processor 4 performs these controls in synchronization. The slit scanning performed in this manner enables acquisition of a high-quality fundus image with high contrast, free from the influence of unnecessary scattered light, by the use of a simple configuration.

In some embodiment examples, a black spot may be provided between the optical scanner 30 and the hole mirror 45 to eliminate harmful reflected light. The black spot is disposed at a position substantially optically conjugate with the position of the central ghost caused by the reflection of the slit light generated by the objective lens 46.

Existing ophthalmic apparatuses of similar types are provided with a focus indicator projection optical system and a mechanism. The focus indicator projection optical system is configured to generate a focus adjustment indicator (split indicator) to be projected onto the fundus Ef, and is disposed between the optical scanner and the hole mirror. The mechanism is configured to move the focus indicator projection optical system. Such existing ophthalmic apparatuses of similar types is operated to project light output from the focus indicator optical system (focus indicator light) onto the fundus Ef, detect fundus reflected light of the projected light by the use of the photographing optical system and the imaging device, identify the position of the split indicator in the acquired image based on Scheiner's principle, and perform focus adjustment. This focus adjustment is performed by moving both the focus indicator projection optical system and the focus lens of the photographing optical system in the respective directions along their corresponding optical axes, based on the identified position of the split indicator. As described above, both the focus indicator projection optical system and the moving mechanism provided in existing ophthalmic apparatuses of similar types, are dedicated hardware components for focus adjustment.

In contrast, the ophthalmic apparatus 1 of the first embodiment example does not include the focus indicator projection optical system and the mechanism for moving the focus indicator projection optical system that are dedicated hardware components for focus adjustment provided in existing ophthalmic apparatuses of similar types. In place of these dedicated hardware components, the ophthalmic apparatus 1 performs focus adjustment by introducing a novel technique described later. This novel technique utilizes a hardware component that is provided with existing ophthalmic apparatuses of similar types in a new way. It should be noted that novel techniques according to some aspect examples may be implemented without a dedicated hardware component for focus adjustment, and that novel techniques according to some other aspect examples may be implemented without complex and large-scale dedicated hardware components for focus adjustment such as the focus indicator projection optical system and the moving mechanism described above.

The processor 4 is configured to perform various kinds of control processes and various kinds of computational processes. For example, the processor 4 is configured to perform at least slit light projection control, a condition determination process, and focus adjustment control. The slit light projection control, the condition determination process, and the focus adjustment control are included in a series of processes for focus adjustment (in particular, autofocusing). By employing a configuration of performing such a series of processes, in other words, by introducing a configuration with a novel software component, the ophthalmic apparatus 1 can perform focus adjustment without using a dedicated hardware component for focus adjustment or without using a complex and large-scale dedicated hardware component for focus adjustment.

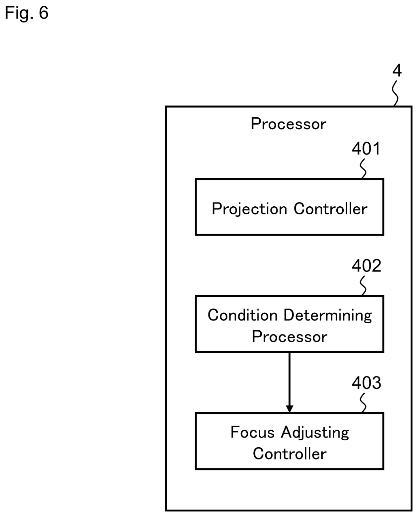

A non-limiting example of the configuration of the processor 4 is shown in FIG. 6. The processor 4 in the present example includes the projection controller 401, the condition determining processor 402, and the focus adjusting controller 403.

The slit light projection control is a process of controlling the imaging unit 2 performed to project slit light for focus adjustment onto the fundus Ef of the subject's eye E. The slit light projection control is performed by the projection controller 401. The mode or aspect of the slit light projection control may be freely selected or determined. Several non-limiting examples of the slit light projection control will be described later.

The condition determination process is a computational process performed to determine a focus adjustment condition based on an output from the imaging device 50 that has detected return light of the slit light projected onto the fundus Ef under the slit light projection control. The condition determination process is performed by the condition determining processor 402. The mode or aspect of the condition determination process may be freely selected or determined. Several non-limiting examples of the condition determination process will be described later.

The focus adjustment control is a process of controlling the focus adjusting unit 3 that is performed to adjust the focus state of the imaging unit 2 based on the focus adjustment condition determined by the condition determination process. The focus adjustment control is performed by the focus adjusting controller 403. The mode or aspect of the focus adjustment control may be freely selected or determined. Several non-limiting examples of the focus adjustment control will be described later.

In the example shown in FIG. 2, the focus adjusting unit 3 includes, from among the components of the imaging unit 2, the moving mechanism 22M that moves the slit aperture diaphragm 22, and the moving mechanism 47M that moves the focus lens 47.

In the case of FIG. 2, the condition determination process is performed to determine a control condition used for the moving mechanism 22M and a control condition used for the moving mechanism 47M. The control condition used for the moving mechanism 22M is determined to perform focus adjustment of the illumination optical system 20, that is, to perform adjustment of the focal position of the illumination optical system 20. The control condition used for the moving mechanism 47M is determined to perform focus adjustment of the photographing optical system 40, that is, to perform adjustment of the focal position of the photographing optical system 40. The control condition used for the moving mechanism 22M includes information indicating the direction and the amount (distance) of the movement of the slit aperture diaphragm 22. The control condition used for the moving mechanism 47M includes information indicating the direction and the amount (distance) of the movement of the focus lens 47. Any of the two control conditions may include other information equivalent to the movement direction and the movement amount of its corresponding component. For example, the control condition used for the moving mechanism 22M (or used for the moving mechanism 47M) may include information indicating the content of a control signal (e.g., the number of pulses in a control pulse signal) to be sent to the moving mechanism 22M (or the moving mechanism 47M).

Furthermore, the focus adjustment control is performed to control the moving mechanism 22M based on the control condition used for the moving mechanism 22M determined by the condition determination process, and also to control the moving mechanism 47M based on the control condition used for the moving mechanism 47M determined by the condition determination process.

In some aspect examples, the focus adjustment of the photographing optical system 40 is performed by moving the focus lens 47 as described herein; however, in other aspect examples, the focus adjustment of the photographing optical system 40 may be performed by moving the imaging device 50 (the image sensor 51). In the latter case, a moving mechanism is provided to move the imaging device 50 (the image sensor 51), and the condition determination process is performed to determine a control condition for this moving mechanism. Based on this control condition, the focus adjustment control then is performed to control this moving mechanism to move the imaging device 50 (the image sensor 51).

In some aspect examples, the projection controller 401 first controls the optical scanner 30 to fix the deflection direction of the slit light in a predetermined direction. In other words, the projection controller 401 maintains the orientation of the reflecting surface (mirror surface) of the optical scanner 30 in a predetermined orientation.

In a state in which the operation of the optical scanner 30 (the operation of changing the orientation of the mirror surface) is stopped in this manner, the projection controller 401 controls the imaging unit 2 to project the slit light onto the fundus Ef. The imaging unit 2 detects the slit light projected onto the fundus Ef in a state in which the operation of the optical scanner 30 is stopped, using the photographing optical system 40 and the imaging device 50. The image obtained in this way is input to the condition determining processor 402.

The condition determining processor 402 determines the focus adjustment condition by analyzing this image. More specifically, by analyzing the image acquired in the state in which the operation of the optical scanner 30 is stopped, the condition determining processor 402 performs a process of identifying the position of the image of the slit light (i.e., the slit light image corresponding to this slit light) projected onto the fundus Ef in the state in which the operation of the optical scanner 30 is stopped, and a process of determining the focus adjustment condition based on the position of the slit light image identified. Several non-limiting examples of these processes performed by the condition determining processor 402 will be described later.

In some aspect examples, the projection controller 401 controls the imaging unit 2 to output at least two slit lights that are projected onto mutually different locations (mutually different areas, mutually different regions) on the fundus Ef of the subject's eye E. Described below is the case of using two slit lights (referred to as the first slit light and the second slit light). However, it will be understood by those skilled in the art that similar procedures can be applied in the case of using three or more slit lights. The first slit light and the second slit light may be output, for example, in a sequential manner or in a simultaneous manner.

The method of generating the first slit light and the second slit light that are projected onto mutually different locations on the fundus Ef of the subject's eye E, may be freely selected or determined. Some aspect examples may utilize the iris diaphragm 21 having two apertures 21A and 21B (referred to as the first aperture 21A and the second aperture 21B) as shown in FIG. 3. Note that these aspect examples may be performed, for example, in a state in which the deflection direction of the slit light by means of the optical scanner 30 is fixed in a predetermined direction (i.e., in a state in which the orientation of the mirror surface of the optical scanner 30 is maintained in a predetermined orientation).

In some examples, the slit light generated based on the light that has passed through the first aperture 21A may be used as the first slit light, and the slit light generated based on the light that has passed through the second aperture 21B may be used as the second slit light. As a result, it becomes possible to generate the first slit light and the second slit light whose projection positions on the fundus Ef of the subject's eye E are different from each other.

In one configuration example that can be adopted for this purpose, the first shutter to open and close the first aperture 21A and the second shutter to open and close the second aperture 21B are provided. Under the control of the projection controller 401, these two shutters are operated to alternately open and close the first aperture 21A and the second aperture 21B, thereby sequentially generating the first slit light and the second slit light that are projected onto mutually different locations on the fundus Ef of the subject's eye E. For example, the first slit light can be generated selectively by having the first aperture 21A open while keeping the second aperture 21B closed. Conversely, the second slit light can be generated selectively by having the second aperture 21B open while keeping the first aperture 21A closed. In addition, by having both apertures 21A and 21B open, both the first slit light and the second slit light can be generated simultaneously.

The first shutter and the second shutter used in the present aspect example are significantly simpler and more compact compared to the dedicated hardware components for focus adjustment (such as the focus indicator projection optical system and the mechanism of moving the same) provided in existing ophthalmic apparatuses of similar types.

In another configuration example, the light source 10 includes the first light source and the second light source. The first light source emits light that passes only through the first aperture 21A, and the second light source emits light that passes only through the second aperture 21B. Under the control of the projection controller 401, the first light source and the second light source may be alternately turned on, thereby sequentially generating the first slit light and the second slit light that are projected onto mutually different locations on the fundus Ef of the subject's eye E. For example, the first slit light can be generated selectively by having the first light source being turned on while keeping the second light source turned off. Conversely, the second slit light can be generated selectively by having the second light source being turned on while keeping the first light source turned off. In addition, having both the first light source and the second light source turned on can simultaneously generate the first slit light and the second slit light.

The first light source and the second light source used in the present aspect example are significantly simpler and more compact compared to the dedicated hardware components for focus adjustment (such as the focus indicator projection optical system and the mechanism of moving the same) found in existing ophthalmic apparatuses of similar types.

In some other aspect examples for generating the first slit light and the second slit light, the optical scanner 30 can be utilized. In the present aspect example, the projection controller 401 generates the first slit light by orienting the mirror surface of the optical scanner 30 to face the predetermined first direction, and generates the second slit light by orienting the mirror surface of the optical scanner 30 to face the predetermined second direction. In this way, the present aspect example can generate the first slit light and the second slit light that are projected onto mutually different locations on the fundus Ef of the subject's eye E. The present aspect example is capable of generating the first slit light and the second slit light without having to additionally provide new hardware components.

By means of any of the methods and techniques described above, or by means of another method, the projection controller 401 controls the imaging unit 2 to output the first slit light and the second slit light that are projected onto mutually different locations on the fundus Ef of the subject's eye E.

The imaging unit 2 obtains the first image and the second image by means of the photographing optical system 40 and the imaging device 50. Here, the first image is an image in which a projection image of the first slit light (first slit light image) on the fundus Ef of the subject's eye E is depicted, and the second image is an image in which a projection image of the second slit light (second slit light image) on the fundus Ef of the subject's eye E is depicted. In the case where the first slit light and the second slit light are generated in a sequential manner, that is, in the case where projection of the first slit light and projection of the second slit light onto the fundus Ef of the subject's eye E are performed separately from each other, the first image and the second image are separate images. On the other hand, in the case where the first slit light and the second slit light are generated in a simultaneous manner, that is, in the case where projection of the first slit light and projection of the second slit light onto the fundus Ef of the subject's eye E are performed simultaneously, the first image and the second image are the same image.

The condition determining processor 402 detects the first slit light image from the first image and obtains the first position information indicating the position of the first slit light image in the first image. Similarly, the condition determining processor 402 detects the second slit light image from the second image and obtains the second position information indicating the position of the second slit light image in the second image. For example, the first position information may be one or more coordinates represented using a coordinate system defined for the first image (e.g., a coordinate system representing pixel positions of the first image), and the second position information may be one or more coordinates represented using a coordinate system defined for the second image (e.g., a coordinate system representing pixel positions of the second image).

The condition determining processor 402 determines the focus adjustment condition based on the relative position between the first slit light image and the second slit light image. More specifically, the condition determining processor 402 may be configured to calculate the difference between the coordinate of the first slit light image (the first coordinate) indicated by the first position information and the coordinate of the second slit light image (the second coordinate) indicated by the second position information, and then determine the focus adjustment condition based on this coordinate difference calculated.

Next, the principle of the focus adjustment according to the first embodiment example and several non-limiting specific examples thereof will be described.

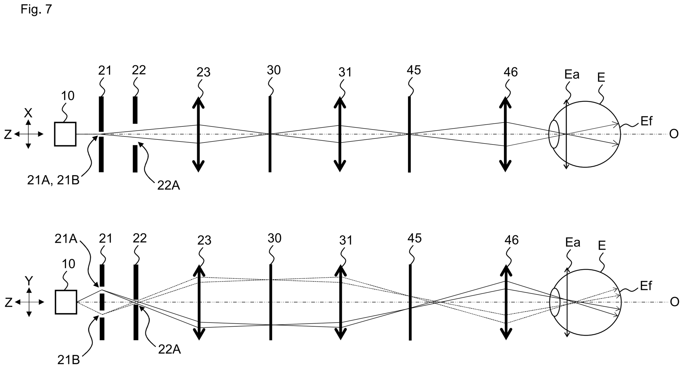

To begin with, the optical path of the illumination optical system 20 that projects the slit light used for focus adjustment onto the fundus Ef of the subject's eye E, will be described. FIG. 7 illustrates a non-limiting example of the optical path formed by the optical systems shown in FIG. 2 through FIG. 4. The upper part of FIG. 7 is a plan view, and the lower part is a side view. Each optical path shown in FIG. 7 is an optical path in a case where the two apertures 21A and 21B of the iris diaphragm 21 are considered as object point positions.

The light emitted by the light source 10 illuminates the iris diaphragm 21. A part of the light that has passed through the first aperture 21A of the iris diaphragm 21 (referred to as the first light) passes through the slit 22A of the slit aperture diaphragm 22, thereby generating the first slit light. Similarly, a part of the light that has passed through the second aperture 21B of the iris diaphragm 21 (referred to as the second light) passes through the slit 22A of the slit aperture diaphragm 22, thereby generating the second slit light.

Here, the first light passing through the first aperture 21A may be, for example, light that has passed through the first aperture 21A when the aforementioned first shutter is in an open state, or light that has been emitted from the aforementioned first light source and passed through the first aperture 21A. Similarly, the second light passing through the second aperture 21B may be, for example, light that has passed through the second aperture 21B when the aforementioned second shutter is in an open state, or light that has been emitted from the aforementioned second light source and passed through the second aperture 21B.

In the illumination optical system 20, the iris diaphragm 21 (the first aperture 21A and the second aperture 21B), the optical scanner 30, and the hole mirror 45 are disposed in a positional relationship that is substantially optically conjugate with each other. In a state where alignment is appropriately established, these components of the illumination optical system 20 are disposed at a position substantially optically conjugate with the anterior segment Ea (e.g., the pupil).

The first slit light generated by the iris diaphragm 21 and the slit aperture diaphragm 22 is relayed by the relay lens 23 to form an image on the mirror surface of the optical scanner 30, and then the first slit light is deflected by this mirror surface. The first slit light deflected by the optical scanner 30 is relayed by the relay lens 31 to form an image on the reflecting part (the mirror part) of the hole mirror 45, and then the first slit light is deflected by the reflecting part. The first slit light deflected by the hole mirror 45 is converted into convergent light by the objective lens 46 and enters the subject's eye E. Then, the first slit light forms an image once at the anterior segment Ea (e.g., the pupil) and is projected onto the fundus Ef. Performing imaging by the use of the photographing optical system 40 and the imaging device 50 when the first slit light is being projected onto the fundus Ef can yield an image (the first image mentioned above) depicting the first slit light image corresponding to the first slit light.

Similarly, by performing imaging using the photographing optical system 40 and the imaging device 50 when the second slit light generated by the iris diaphragm 21 and the slit aperture diaphragm 22 is being projected onto the fundus Ef, an image (the second image mentioned above) depicting the second slit light image corresponding to the second slit light can be obtained.

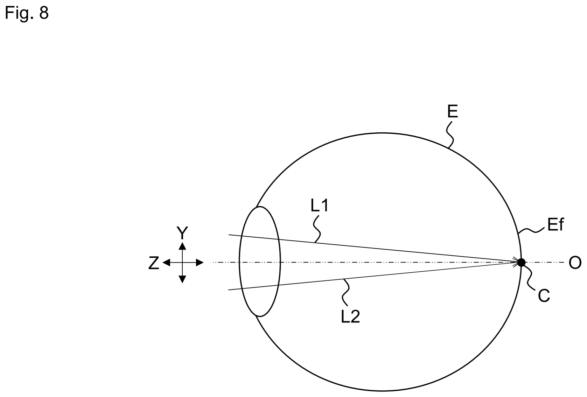

When the fundus Ef is in focus of the illumination optical system 20, as shown in FIG. 8, the first slit light L1 and the second slit light L2 are projected onto substantially the same position on the fundus Ef. In other words, when the fundus Ef is in focus of the illumination optical system 20, the intersection position C of the first slit light L1 and the second slit light L2 is located on the fundus Ef.

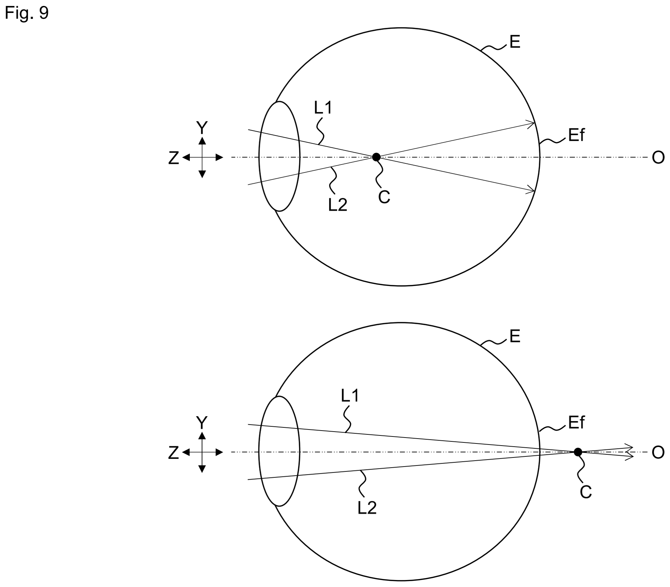

On the other hand, when the fundus Ef is not in focus of the illumination optical system 20, as shown in FIG. 9, the position on the fundus Ef onto which the first slit light L1 is projected and the position on the fundus Ef onto which the second slit light L2 is projected are differs from each other. In other words, when the fundus Ef is not in focus of the illumination optical system 20, the intersection position C of the first slit light L1 and the second slit light L2 is located at a position away from the fundus Ef.

Note that the upper diagram in FIG. 9 illustrates a state in which the intersection position C of the first slit light L1 and the second slit light L2 is located in front of the fundus Ef (i.e., toward the anterior segment Ea), representing a so-called "front-focused" state relative to the fundus Ef. Conversely, the lower diagram shows a state in which the intersection position C of the first slit light L1 and the second slit light L2 is located behind the fundus Ef, representing a so-called "back-focused" state relative to the fundus Ef.

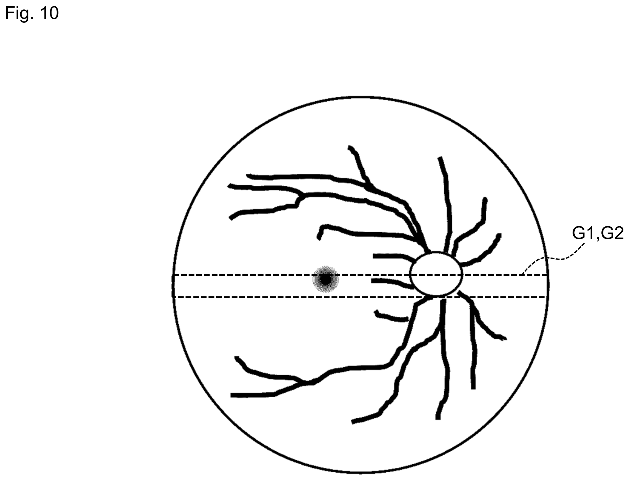

When the fundus Ef is in focus of the illumination optical system 20 as shown in FIG. 8, the first slit light L1 and the second slit light L2 are projected onto substantially the same position on the fundus Ef. An example of a fundus image acquired in this case is shown in FIG. 10. In the fundus image of FIG. 10, the first slit light image G1 and the second slit light image G2 are depicted at substantially the same position. Here, the first slit light image G1 is a fundus projection image of the first slit light L1, and the second slit light image G2 is a fundus projection image of the second slit light L2.

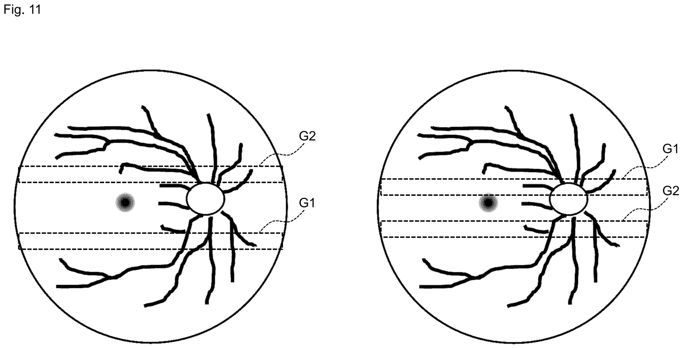

In contrast, when the fundus Ef is not in focus of the illumination optical system 20 as shown in FIG. 9, the first slit light L1 and the second slit light L2 are projected onto different positions on the fundus Ef. An example of a fundus image acquired in this case is shown in FIG. 11.

The fundus image on the left side in FIG. 11 is an image obtained in the case of the "front-focused" state illustrated in the upper diagram of FIG. 9. In this fundus image, the first slit light image G1 that represents a fundus projection image of the first slit light L1 and the second slit light image G2 that represents a fundus projection image of the second slit light L2 are depicted at different positions from each other. More specifically, the first slit light image G1 is depicted below the central position in the vertical direction of the frame of the fundus image, and the second slit light image G2 is depicted above the central position.

On the other hand, the fundus image on the right side in FIG. 11 is an image obtained in the case of "back-focused" state illustrated in the lower diagram of FIG. 9. In this fundus image, the first slit light image G1 that represents a fundus projection image of the first slit light L1 and the second slit light image G2 that represents a fundus projection image of the second slit light L2 are depicted at different positions from each other. More specifically, the first slit light image G1 is depicted above the central position in the vertical direction of the frame of the fundus image, and the second slit light image G2 is depicted below the central position.

As described above, according to the first embodiment example, it becomes possible to determine whether the focus state of the imaging unit 2 relative to the fundus Ef of the subject's eye E is appropriate or not by utilizing existing hardware components provided in conventional fundus imaging modalities of slit-scanning type. The first embodiment example configured in this way does not require a (complex and large-scale) dedicated hardware component for focus adjustment, such as those found in conventional ophthalmic apparatuses of similar types.

Furthermore, according to the first embodiment example, it is possible to determine the direction of defocus of the imaging unit 2 relative to the fundus Ef. In other words, the first embodiment example is capable of determining whether the focus state of the imaging unit 2 is front-focused or back-focused.

In addition, the first embodiment also enables determination of the amount or degree of defocus of the imaging unit 2 relative to the fundus Ef. This will be detailed later.

Thus, according to the first embodiment example, without employing a complex and large-scale dedicated hardware component for focus adjustment as found in conventional ophthalmic apparatuses of similar types, it becomes feasible to perform autofocusing of the imaging unit 2 relative to the fundus Ef of the subject's eye E by utilizing the hardware components already present in existing slit-scanning type fundus imaging modalities.

As will be elaborated upon later, in some aspect examples, the first image in which the first slit light image G1 is depicted and the second image in which the second slit light image G2 is depicted can be acquired by separately projecting the first slit light L1 and the second slit light L2 onto the fundus Ef, and then a parameter related to the focus state (e.g., the direction and/or amount of defocus) can be identified by comparing the two images G1 and G2.

In some other aspect examples, a single image can be obtained by simultaneously projecting the first slit light L1 and the second slit light L2, whose characteristics (e.g., wavelengths, intensities (amounts of light)) are different from each other, onto the fundus Ef. Based on the relative position between the first slit light image G1 and the second slit light image G2 in the single image, a parameter related to the focus state (e.g., the direction and/or amount of defocus) can be determined. It should be noted that the first slit light L1 and the second slit light L2 having mutually different characteristics may be projected onto the fundus Ef in the case where the first slit light L1 and the second slit light L2 are projected separately onto the fundus Ef.

The condition determining processor 402 determines a focus adjustment condition based on the aforementioned principle described with reference to FIG. 8 through FIG. 11. The condition determining processor 402 is configured to determine the focus adjustment condition based on an output (e.g., the first image, the second image) from the imaging device 50 that has detected the return light of the slit light (e.g., the first slit light L1, the second slit light L2) projected onto the fundus Ef of the subject's eye E under the slit light projection control.

In some aspect examples, the condition determining processor 402 may be configured to determine the focus adjustment condition based on the position of the slit light image (e.g., the first slit light image G1, the second slit light image G2) in the image (e.g., the first image, the second image) generated by the imaging device 50 that has detected the return light of the slit light (e.g., the first slit light L1, the second slit light L2) projected onto the fundus Ef of the subject's eye E under the slit light projection control.

The method for determining the position of the slit light image in the fundus image may be freely selected or determined. The condition determining processor 402 of some aspect examples determines the position of the slit light image based on an intensity profile in an image region that is at least a part of the slit light image formed along the first direction corresponding to the direction of the movement of the projection position of the slit light projected by the imaging unit 2.

In some aspect examples, the slit length direction of the slit 22A of the slit aperture diaphragm 22 corresponds to the X direction, and the movement direction (scanning direction) of the projection position of the slit light is the Y direction. In the fundus image acquired by the imaging unit 2, the direction corresponding to the X direction is also referred to as the X direction, and the direction corresponding to the Y direction is also referred to as the Y direction. The longitudinal direction of the slit light image depicted in the fundus image is the X direction.

The condition determining processor 402 first generates an intensity profile of the slit light image. This intensity profile may be defined for the entire slit light image, or may be defined for an image region that is a part of the slit light image. In addition, as a preparatory step for generating the intensity profile, the condition determining processor 402 may apply image segmentation to at least a part of the fundus image to identify the slit light image in the fundus image, thereby determining an area (region) to which the intensity profile generation process is applied. The intensity profile generated by the condition determining processor 402 represents a distribution of brightness in the first direction (the Y direction in the fundus image) corresponding to the scanning direction (the Y direction in real space).

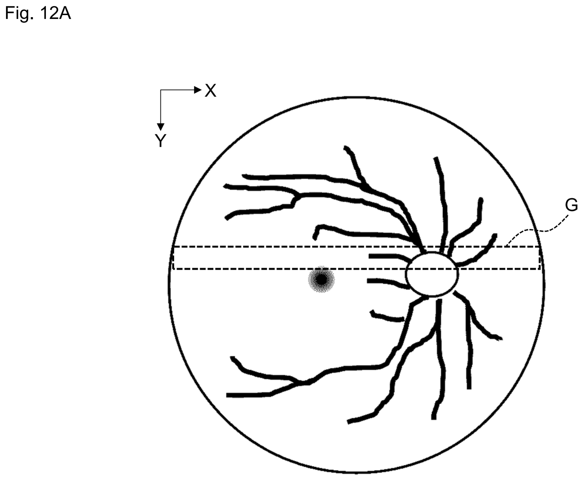

A case of generating an intensity profile of the slit light image G depicted in the fundus image shown in FIG. 12A will now be described. In FIG. 12A, the X direction (+X direction) is the rightward direction, and the Y direction (+Y direction) is the downward direction.

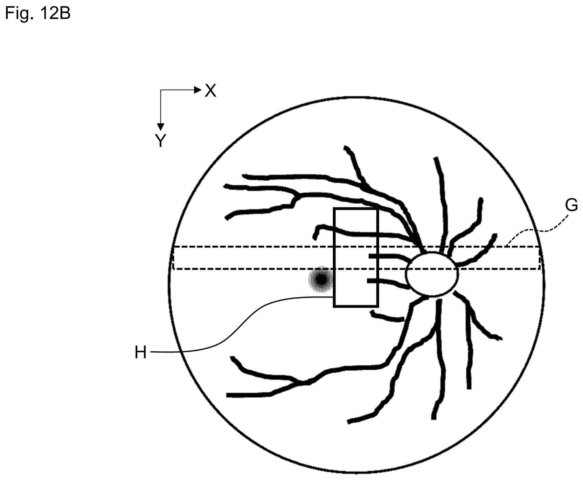

The condition determining processor 402 first designates or determines the analysis region H in the fundus image used for intensity profile generation (refer to FIG. 12B). As previously mentioned, the analysis region H may be determined by the use of image segmentation or may be designated or determined at a predetermined location.

In the analysis region H of some examples, a plurality of pixels is arranged in both the X direction and the Y direction (that is, arranged in a two-dimensional array). In other words, the analysis region H includes a plurality of pixel columns extending in the Y direction, and these plurality of pixel columns are arranged in the X direction. The condition determining processor 402 may generate an intensity profile along the Y direction by summing, in the X direction, the intensity values of the plurality of pixels arranged in the two-dimensional array in the analysis region H.

In some other aspect examples, a linear analysis region (one-dimensional analysis region) along the Y direction may be designated or determined, and an intensity profile may be generated for this one-dimensional analysis region. While this method offers simplicity as the advantage, it has the drawback that any noise present within the one-dimensional analysis region directly affects the resulting intensity profile. Therefore, the method utilizing a two-dimensional analysis region (H) provides the advantage of being able to reduce the effect of noise. Furthermore, according to the method utilizing a two-dimensional analysis region (H), the difference between the brightness of the slit light image G and the brightness of other image regions (typically, the former is brighter, and the latter is dimmer) can be emphasized, making it possible to improve the quality (e.g. precision, accuracy, reproducibility) of the resulting intensity profile.

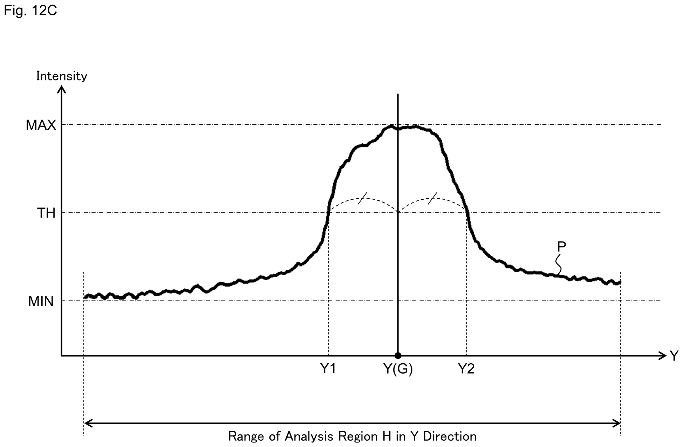

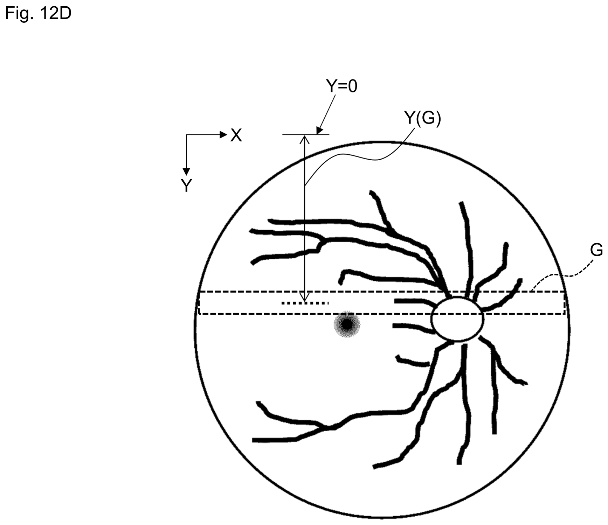

An example of the intensity profile of the slit light image G is shown in FIG. 12C. In some aspect examples, the condition determining processor 402 calculates the maximum value (MAX) and the minimum value (MIN) of the intensity profile P, and then calculates their midpoint (intermediate) value TH as follows: TH = (MAX - MIN) / 2. The condition determining processor 402 then determines an intersection point between the intensity profile P and the straight line defined by "intensity = TH". There are two such intersection points. The Y coordinates of the two intersection points are designated as Y1 and Y2. The condition determining processor 402 determines the midpoint (intermediate) value Y(G) between the Y coordinates Y1 and Y2 of the two identified intersections: Y(G) = abs (Y1 - Y2) / 2. Here, the notation "abs (α)" represents the absolute value of the value α. In the present aspect example, the value Y(G) obtained in this way is adopted as the position (representative position, centroid position) of the slit light image G (see FIG. 12D).

The method for determining the position of the slit light image is not limited to the above method. For example, while the above method calculates the midpoint (intermediate) value TH (TH = (MAX - MIN) / 2) between the maximum value (MAX) and the minimum value (MIN) of the intensity profile, more generally, the formula "TH = (MAX - MIN) / R" may be employed. Here, the value R may be a real number determined in advance, or a real number determined based on the intensity profile (or information equivalent thereto).

In some aspect examples, the position of the slit light image may be determined by considering the area under the curve (AUC) of the intensity profile. For instance, when the area under the curve of the entire intensity profile is denoted as A, the Y coordinate that divides the area under the curve A into subareas at a predetermined ratio may be determined, and this Y coordinate may be adopted as the position (representative position, centroid position) of the slit light image. As a specific example, the Y coordinate that divides the area under the curve A of the entire intensity profile into two subareas at a 1:1 ratio may be determined and used as the position of the slit light image.

In some other aspect examples, the value TH may be a fixed value. The method for determining this fixed value TH may be freely selected or determined. For example, the fixed value TH may be derived by applying statistical computations to a large amount of clinically collected data, may be derived based on data obtained from measurements conducted using a model eye, may be derived by using a computer simulation such as ray tracing, or may be derived by the use of freely selected combination of these methods. Alternatively, the fixed value TH may be determined by using any other methods.

The condition determining processor 402 determines the focus adjustment condition based on the position information of the slit light image obtained in this manner.

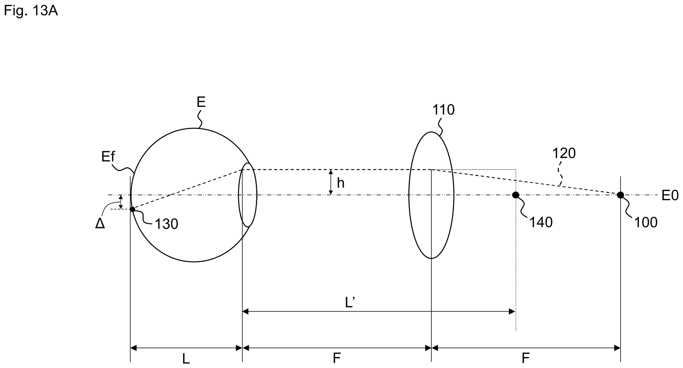

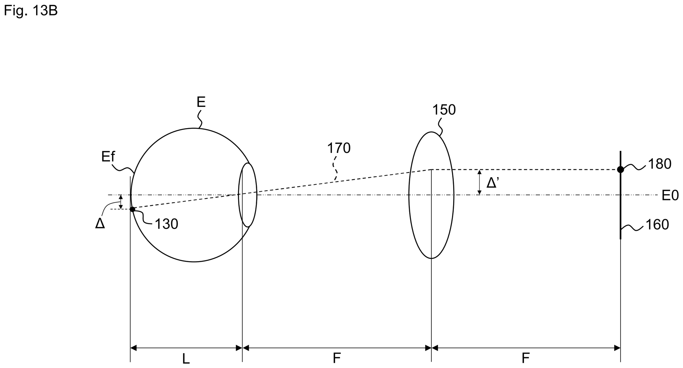

First, the principle of the process performed to determine the focus adjustment condition (e.g., the adjustment amount for the position of the slit aperture diaphragm 22 and the adjustment amount for the position of the focus lens 47) based on the position (Y coordinate) of the slit light image will be described with further reference to FIG. 13A and FIG.13B.

FIG. 13A illustrates the path of light projected onto the fundus Ef of the subject's eye E by the illumination optical system. The reference character 100 denotes a predetermined target disposed on the optical axis E0 of the subject's eye E. The reference character 110 represents the optical system located between the target 100 and the subject's eye E. The focal length of the optical system 110 is denoted as F. The axial length of the subject's eye E is represented as L, and its refractive power as D.

Consider the case where light (parallel light) 120 emitted from the target 100 passes through the optical system 110 and is projected onto the subject's eye E. Let h denote the height (distance from the optical axis E0) of the light 120 entering the subject's eye E. The light 120 entering the subject's eye E is refracted by the ocular optical system (cornea, crystalline lens, etc.) and then projected onto the position 130 on the fundus Ef.

Here, in the optical system illustrated in FIG. 2 to FIG. 4, the height h of the light 120 is half the value of the distance between the two apertures 21A and 21B of the iris diaphragm 21 that are disposed at positions substantially optically conjugate with the pupil of the subject's eye E.

When the subject's eye E is normal vision (0 diopters), the projection position 130 of the light 120 on the fundus Ef is located at the center of the fundus Ef (i.e., located on the optical axis E0). In contrast, when the subject's eye E is not normal vision, the projection position 130 of the light 120 on the fundus Ef is shifted (displaced) from the optical axis E0 by the distance Δ (where positional shift amount Δ > 0).

Let the optically conjugate point with the fundus Ef corresponding to the refractive power D of the subject's eye E be denoted as the reference character 140. The distance from the subject's eye E to the conjugate point 140 is denoted as L', where L' = 1000 / D. Then, as can be seen from FIG. 13A, Δ / L = h / L', and therefore Δ = L × h / L'. As mentioned above, the height h of the light 120 is a fixed value determined on the basis of the iris diaphragm 21. Then, the positional shift amount Δ of the projection position 130 of the light 120 on the fundus Ef relative to the optical axis E0 of the subject's eye E, varies depending on the refractive power D of the subject's eye E (i.e., the distance L' between the subject's eye E and the conjugate point 140) and on the axial length L of the subject's eye E. Note that even when the target 100 has a ring shape or an arc shape, the positional shift amount of the projection position 130 of the light 120 on the fundus Ef relative to the optical axis E0 of the subject's eye E is represented by Δ.

FIG. 13B illustrates the path of light when imaging of a target image (a projected image of the target 100) projected onto the fundus Ef at a position shifted by the distance Δ from the optical axis E0 of the subject's eye E is performed using a photographing optical system arranged coaxially with the optical axis E0. The reference character 160 denotes the imaging surface (imaging surface of the imaging device 50), and the reference character 150 represents the optical system located between the subject's eye E and the imaging surface 160. The focal length of this optical system 150 is denoted as F, identical to that of the optical system 110 in FIG. 13A.

When the subject's eye E is normal vision, the light 170 leaving from the projection position 130 on the fundus Ef passes through the optical system 150 and then forms an image at the intersection position of the imaging surface 160 and the optical axis E0. On the other hand, when the subject's eye E is not normal vision, the image position (image formation position) of the light 170 on the imaging surface 160 is a position shifted from the optical axis E0 by the distance Δ' (where positional shift amount Δ' > 0). In this case, the image position in the direction along the optical axis E0 (Z direction) also shifts.

As can be seen from FIG. 13B, since the relationship Δ / L = Δ' / F holds, it follows that Δ' = F × Δ / L. Given that Δ = L × h / L' as mentioned above, it follows that Δ' = F × h / L'. Furthermore, since L' = 1000 / D, it follows that Δ' = F × h × D / 1000.

Therefore, the refractive power D of the subject's eye E for a specific meridian is expressed by the following equation: D = (1000 × Δ') / (F × h). Here, the value F is the focal length of the photographing optical system, and the value h is half the value of the distance between the two apertures 21A and 21B of the iris diaphragm 21, both of which are known values. Consequently, by obtaining the positional shift amount Δ' of the light 170 on the imaging surface 160, the refractive power D of the subject's eye E at a specific meridian can be determined.

Existing ophthalmic apparatuses of similar types to the ophthalmic apparatus 1 according to the first embodiment example have, in advance, information representing the relationship between refractive powers of subject's eyes and adjustment amounts (movement distances) of the optical components for focus adjustment. An example of the optical components for focus adjustment is the focus indicator projection optical system and the focus lens described above. Further, the existing ophthalmic apparatuses of similar types to the ophthalmic apparatus 1 according to the first embodiment example refers to this information to perform focus adjustment corresponding to the refractive power of each subject's eye.

Similarly, the ophthalmic apparatus 1 of some aspect examples may have, in advance, information representing the relationship between refractive powers of subject's eyes and adjustment amounts (movement distances) of the optical components for focus adjustment. In the optical system shown in FIG. 2 to FIG. 4, the slit aperture diaphragm 22 and the focus lens 47 correspond to the optical components for focus adjustment. By referring to this information, the ophthalmic apparatus 1 may determine the focus adjustment condition based on the position of the slit light image in the image generated by the imaging device 50. The focus adjustment condition of this example may include the movement direction and the movement amount (or information corresponding thereto) of the slit aperture diaphragm 22, and the movement direction and the movement amount (or information corresponding thereto) of the focus lens 47.

The ophthalmic apparatus 1 of some other aspect examples may have, in advance, information representing the relationship between relative positions (distances in the Y direction) of the two slit light images and adjustment amounts of the optical components for focus adjustment. In this case, by referring to this information, the ophthalmic apparatus 1 may determine the focus adjustment condition based on the position of the slit light image in the image generated by the imaging device 50.

More generally, the ophthalmic apparatus 1 according to the first embodiment example may be configured to determine the focus adjustment condition based on freely selected or determined information obtainable from information (typically, an image) generated by performing imaging of the fundus Ef onto which the slit light is projected.

The focus adjusting controller 403 performs control (focus adjustment control) of the focus adjusting unit 3 based on the focus adjustment condition determined by the condition determining processor 402 by means of any of the aforementioned methods. The focus adjustment control of the present example includes the following processes: a process of controlling the moving mechanism 22M based on the movement direction and the movement amount (or information corresponding thereto) of the slit aperture diaphragm 22 included in the focus adjustment condition; and a process of controlling the moving mechanism 47M based on the movement direction and the movement amount (or information corresponding thereto) of the focus lens 47 included in the focus adjustment condition.

Some aspect examples may be configured to drive both the slit aperture diaphragm 22 and the focus lens 47 by the use of a single moving mechanism. In this case, the condition determining processor 402 may be configured to determine a single focus adjustment condition, and the focus adjusting controller 403 may be configured to control this single moving mechanism based on the single focus adjustment condition determined.

In some aspect examples described above, the ophthalmic apparatus 1 performs control of the imaging unit 2 in the slit light projection control such that the slit light to be projected onto the fundus Ef of the subject's eye E is emitted from a position (the aperture 21A or 21B of the iris diaphragm 21) spaced a predetermined distance (height h) from the optical axis of the imaging unit 2. Note that the optical axis O of the imaging unit 2 substantially coincides with the optical axis E0 of the subject's eye E in the state of appropriate alignment.

Furthermore, in the condition determination process, the ophthalmic apparatus 1 determines the focus adjustment condition, based on the predetermined distance (height h), the position of the slit light image (positional shift amount Δ') in the image generated by the imaging device 50 of the imaging unit 2, and the focal length (F) of the imaging unit 2. Here, the focus adjustment condition determined includes: the movement direction and the movement amount of the slit aperture diaphragm 22, or information corresponding thereto; and the movement direction and the movement amount of the focus lens 47, or information corresponding thereto.

Then, the ophthalmic apparatus 1 performs control of the focus adjusting unit 3 (the moving mechanism 22M and the moving mechanism 47M) based on the focus adjustment condition obtained by the condition determination process.

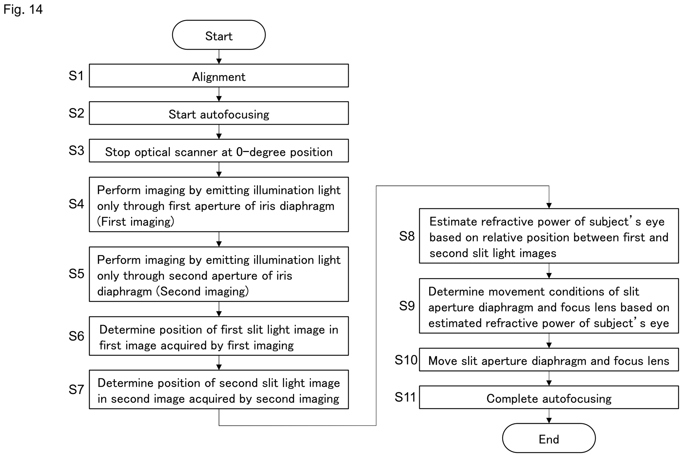

Several non-limiting examples of an operation performed by the ophthalmic apparatus 1 according to the first embodiment example will now be described. FIG. 14 shows one example of an operation for autofocusing performed by the ophthalmic apparatus 1.

Prior to the autofocusing operation, the ophthalmic apparatus 1 performs alignment of the imaging unit 2 relative to the fundus Ef of the subject's eye E (S1). After completing the alignment, the ophthalmic apparatus 1 starts the autofocusing operation (S2). Note that tracking may be performed to maintain the appropriate alignment state achieved by the step S1. Tracking refers to the operation of maintaining the appropriate positional relationship (appropriate alignment state) between the subject's eye E and the imaging unit 2, by moving the optical system of the apparatus in accordance with the movement of the subject's eye E.

In the autofocusing operation of the present example, the projection controller 401 first places the mirror surface of the optical scanner 30 at a predetermined neutral position (0-degree position) (S3). The 0-degree position is, for example, the central position of the movable range of the mirror surface of the optical scanner 30. In the present example, the autofocusing operation is performed in a state where the orientation of the mirror surface of the optical scanner 30 is fixed, that is, in a state where the operation of the optical scanner 30 is stopped.

Next, the projection controller 401 controls the light source 10 and/or the illumination optical system 20 to output illumination light only through one of the two apertures 21A and 21B of the iris diaphragm 21 (for example, only through the first aperture 21A). A part of the illumination light emitted from the first aperture 21A passes through the slit 22A of the slit aperture diaphragm 22 and is converted into slit light (the first slit light). The first slit light is relayed by the relay lens 23, deflected by the stationary optical scanner 30, relayed by the relay lens 31, deflected by the mirror part of the hole mirror 45, refracted by the objective lens 46, enters the subject's eye E, and is projected onto the fundus Ef. The photographing optical system 40 and the imaging device 50 perform imaging of the fundus Ef onto which the first slit light is being projected, thereby generating an image (the first imaging: S4).

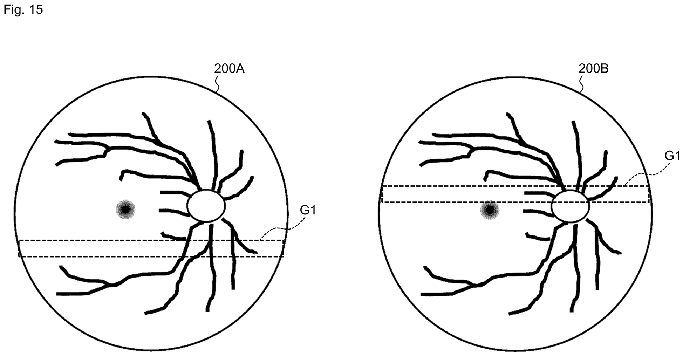

The image obtained by the first imaging in the step S4 is referred to as the first image. Two examples of the first image are illustrated in FIG. 15. The fundus image 200A on the left side in FIG. 15 is an example of the first image acquired when the focus is in a front-focused state, and the slit light image G1 (the first slit light image G1) is depicted at a position below the central position in the Y direction of the fundus image 200A. Conversely, the fundus image 200B on the right side in FIG. 15 is an example of the first image acquired when the focus is in a back-focused state, and the slit light image G1 (the first slit light image G1) is depicted at a position above the central position in the Y direction of the fundus image 200B.

Subsequently, the projection controller 401 controls the light source 10 and/or the illumination optical system 20 to output illumination light only through the other of the two apertures 21A and 21B of the iris diaphragm 21 (for example, only through the second aperture 21B). A part of the illumination light emitted from the second aperture 21B passes through the slit 22A of the slit aperture diaphragm 22 and is converted into slit light (the second slit light). The second slit light is projected onto the fundus Ef through the same path as that of the first slit light. The photographing optical system 40 and the imaging device 50 perform imaging of the fundus Ef onto which the second slit light is being projected, thereby generating an image (the second imaging: S5).

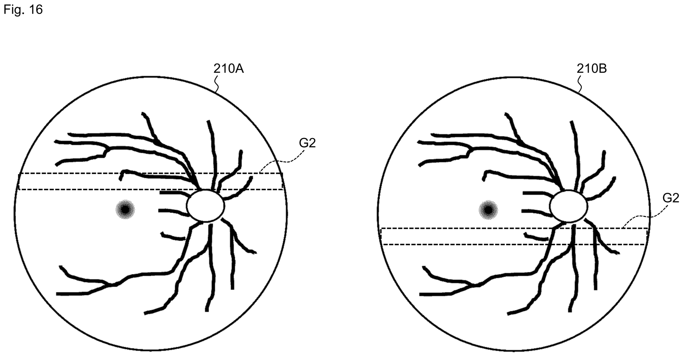

The image obtained by the second imaging in the step S5 is referred to as the second image. Two examples of the second image are illustrated in FIG. 16. The fundus image 210A on the left side in FIG. 16 is an example of the second image acquired when the focus is in a front-focused state, and the slit light image G2 (the second slit light image G2) is depicted at a position above the central position in the Y direction of the fundus image 210A. Conversely, the fundus image 210B on the right side in FIG. 16 is an example of the second image acquired when the focus is in a back-focused state, and the slit light image G2 (the second slit light image G2) is depicted at a position below the central position in the Y direction of the fundus image 210B.

In the present operation example, the second imaging is performed subsequent to the first imaging. However, in another operation example, the second imaging may be performed prior to the first imaging. Furthermore, the number of imaging operations performed for autofocusing is not limited to two (i.e., the first imaging and the second imaging) and may be three or more. It will be readily understood by those skilled in the art that autofocusing may also be performed through a process similar to that of the present operation example, even in cases where three or more imaging operations are carried out. As will be described later, the number of imaging operations performed for autofocusing may also be limited to a single instance.

The first image acquired in the step S4 and the second image acquired in the step S5 are input to the condition determining processor 402.

The condition determining processor 402 may optionally perform registration (position matching) between the first image and the second image. This registration may include, for example, the following processes: a process of detecting a feature point in the first image; a process of detecting a feature point in the second image; a process of computing the positional shift amount between the first image and the second image based on the feature point detected in the first image and the feature point detected in the second image; and a process of adjusting the relative position between the first image and the second image to compensate for (to cancel out, to eliminate) the positional shift amount computed. A feature points used as a reference for registration is a predetermined landmark on the fundus Ef, and may be, for example, a tissue such as the optic nerve head, macula, or blood vessels, or may be a lesion, treatment scar, or the like. The condition determination process may be applied to the first image and the second image to which such registration has been applied. Note that some other aspect examples may be configured to perform a process up to the determination of the positional shift amount between the first image and the second image, and then perform the condition determination process by the use of the positional shift amount.

Next, as a specific example of the processing executed by the condition determining processor 402, a case where the focus is in a front-focused state will be described. It should be noted that similar processing can be executed in the case where the focus is in a back-focused state.

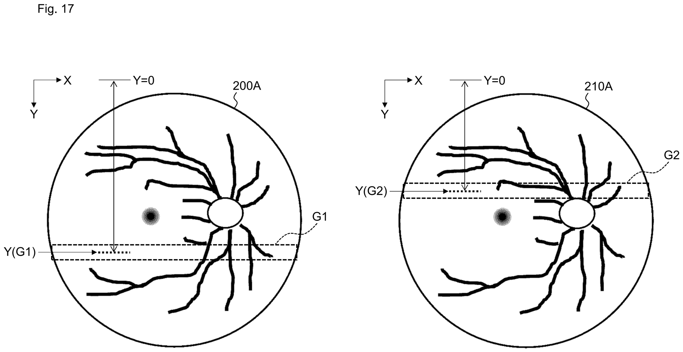

Referring further to FIG. 17, the fundus image 200A on the left side in FIG. 17 is the aforementioned example of the first image acquired by the first imaging in the step S4 when the focus is in the front-focused state, and the fundus image 210A on the right side is the aforementioned example of the second image acquired by the second imaging in the step S5 when the focus is in the front-focused state.

The condition determining processor 402 analyzes the fundus image 200A acquired by the first imaging in the step S4 to detect the first slit light image G1, and then determines the position (Y(G1)) of the first slit light image G1 detected (S6). Similarly, the condition determining processor 402 analyzes the fundus image 210A acquired by the second imaging in the step S5 to detect the second slit light image G2, and then determines the position (Y(G2)) of the second slit light image G2 detected (S7).

Subsequently, the condition determining processor 402 performs estimation of refractive power of the subject's eye E, based on the relative position between the position of the first slit light image G1 determined in the step S6 and the position of the second slit light image G2 determined in the step S7 (S8).

The condition determining processor 402 then determines a movement condition (movement direction and movement amount) of the slit aperture diaphragm 22 and a movement condition (movement direction and movement amount) of the focus lens 47, based on the estimated refractive power of the subject's eye E determined in the step S8 (S9).

The movement directions included in the movement conditions obtained in the step S9 are determined based on the positional relationship between the first slit light image G1 and the second slit light image G2. In the present example, since the first slit light image G1 is located below the second slit light image G2, it is then determined that the focal point is located in front of the fundus Ef (i.e., toward the anterior segment Ea), indicating a front-focused state. On the other hand, when the first slit light image G1 is located above the second slit light image G2, it is determined that the focal point is located behind the fundus Ef, indicating a back-focused state. As previously described, the relationship between the positional relationship of the two slit light images and the shift direction of focal point is known information that can be determined by the design and configuration of the optical system of the imaging unit 2.

Furthermore, the movement amounts included in the movement conditions obtained in the step S9 are determined based on information in which the relationship between refractive powers (diopters) and movement amounts of the optical components for focus adjustment (the slit aperture diaphragm 22 and the focus lens 47 in the present operation example) is recorded. This information is generated in advance and stored, for example, in the memory 5.

The focus adjustment conditions (in the present operation example, the movement condition of the slit aperture diaphragm 22 and the movement condition of the focus lens 47 determined in the step S9) determined by the condition determining processor 402 are transmitted to the focus adjusting controller 403.

The focus adjusting controller 403 performs control of the focus adjusting unit 3 based on the focus adjustment conditions determined in the step S9 (S10). In the present operation example, the focus adjusting controller 403 controls the moving mechanism 22M based on the movement condition of the slit aperture diaphragm 22 determined in the step S9 to move the slit aperture diaphragm 22 by the movement amount according to this movement condition in the movement direction according to this movement condition. In addition, the focus adjusting controller 403 controls the moving mechanism 47M based on the movement condition of the focus lens 47 determined in the step S9 to move the focus lens 47 by the movement amount according to this movement condition in the movement direction according to the movement condition.

Through the processes described above, automatic focus adjustment of the imaging unit 2 (the illumination optical system 20 and the photographing optical system 40) in accordance with the refractive power of the subject's eye E is achieved. That is, both the focal point of the illumination optical system 20 and the focal point of the photographing optical system 40 are automatically disposed at (or in the vicinity of) the fundus Ef of the subject's eye E. Upon completion of this autofocusing (S11), the ophthalmic apparatus 1 may perform imaging (slit scanning) of the fundus Ef.

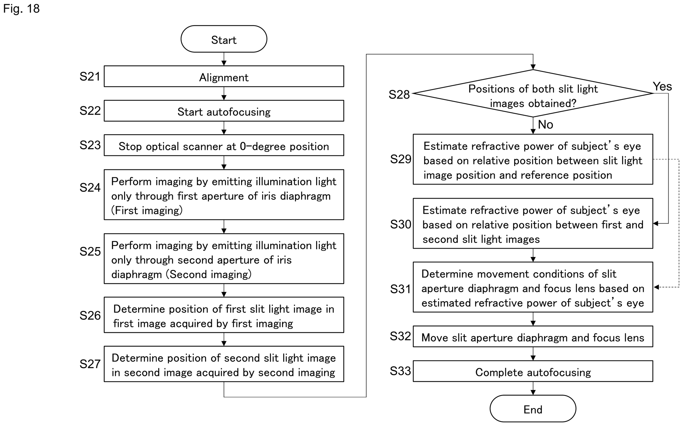

FIG. 18 shows another example of the operation for autofocusing performed by the ophthalmic apparatus 1 according to the first embodiment example.

In the operation example of FIG. 14 described above, the focus adjustment conditions are determined based on the relative position of two slit light images. However, there may be cases in which one of the two slit light images cannot be detected. For example, in a case where the pupil of the subject's eye E is small, either the first slit light or the second slit light may be partially or entirely blocked (vignetted) by the iris, such that no slit light image is formed with respect to the vignetted slit light. Further, in the case where the subject's eye E has a cataract, either the first slit light or the second slit light may be partially or entirely blocked by a clouded region (opacity) of the crystalline lens, such that no slit light image is formed with respect to the blocked slit light, or either the first slit light or the second slit light may be attenuated by the clouded region of the crystalline lens, such that its slit light image becomes unclear. The present operation example of FIG. 18 can provide an autofocusing technique that may also be applied in the cases where such problems occur.

The steps S21 to S27 are performed in the same manner as the steps S1 to S7 in FIG. 14, respectively. Note that, in the steps S26 and S27 of the present operation example, not only the case where the position of the slit light image is detected but also the case where the position of the slit light image is not detected is taken into account.

In the case where the position of the first slit light image is not detected in the step S26, or in the case where the position of the second slit light image is not detected in the step S27 (S28: No), the process proceeds to the step S29. That is, in the case where the detection of the position of the first slit image is unsuccessful in the step S26 but the detection of the position of the second slit light image is successful in the step S27, the process proceeds to the step S29. Similarly, in the case where the detection of the position of the first slit image is successful in the step S26 but the detection of the position of the second slit light image is unsuccessful in the step S27, the process proceeds to the step S29.

In contrast, in the case where the detection of the position of the first slit image is successful in the step S26 and the detection of the position of the second slit light image is also successful in the step S27 (S28: Yes), the process proceeds to the step S30.

Note that, although not illustrated in the drawings, in the case where the detection of the position of the first slit image is unsuccessful in the step S26 and the detection of the position of the second slit light image is also unsuccessful in the step S27, for example, the ophthalmic apparatus 1 may output error information or warning information by the use of the user interface 6. In such a case, the ophthalmic apparatus 1 may restart the focus adjustment (and the alignment, which is the preceding process) from the beginning (i.e., the process may be returned to the step S21 or S23). Alternatively, the ophthalmic apparatus 1 may switch the operation mode from the autofocus mode to a manual focus mode. In the manual focus mode, the user conducts a manual operation to perform focus adjustment by using the user interface 6 while referring to the observation image of the fundus Ef.

In the case where only one of the position detection of the first slit light image and the position detection of the second slit light image is successful (S28: No), the condition determining processor 402 estimates the refractive power of the subject's eye E based on the relative position between the position of the single slit light image detected and a predetermined reference position (S29).

The method of determining the reference position referred to in the step S29 may be freely selected or determined. For example, the reference position may be determined: based on data obtained from measurement using a model eye; by means of a computer simulation such as ray tracing; based on data obtained from measurement of healthy eyes (a healthy eye is an eye that do not have a condition such as small pupil or cataract that could hinder the autofocusing of the present embodiment example); by any combination of these methods; or by using another method. In the method using a model eye, for instance, the position where both slit light images coincide with each other may be determined by performing the steps S23 to S27, and the position determined in this manner may be used as the reference position. Similar procedures may be applied to determine the reference position also in the cases of using other methods.

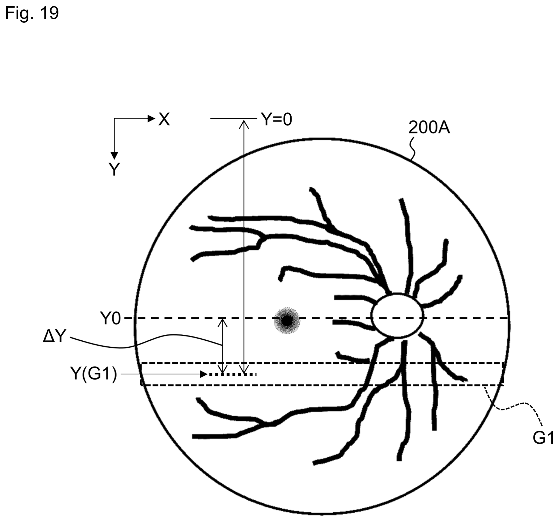

For example, in the case where the slit light image G1 in the fundus image 200A on the left side in FIG. 15 is detected, the condition determining processor 402 determines the position (Y(G1)) of this slit light image G1 as shown in FIG. 19. The condition determining processor 402 then estimates the refractive power of the subject's eye E based on the relative position (ΔY) of the determined position (Y(G1)) of the slit light image G1 relative to the reference position (Y0).

In the case where both the position detection of the first slit light image and the position detection of the second slit light image are successful (S28: Yes), the condition determining processor 402 performs estimation of the refractive power of the subject's eye E in the same manner as in the step S8 of FIG. 14 (S30).

Next, the condition determining processor 402 determines a movement condition (movement direction and movement amount) of the slit aperture diaphragm 22 and a movement condition (movement direction and movement amount) of the focus lens 47, based on the estimated refractive power of the subject's eye E obtained in the step S29 or S30 (S31). Here, the movement directions are determined based on the positional relationship between the slit light image and the reference position. The method of determining the movement amounts may be performed in the same manner as in the step S9 of FIG. 14.

Subsequently, the focus adjusting controller 403 performs control of the focus adjusting unit 3 based on the focus adjustment condition determined in the step S31 (S32). This process is performed in the same manner as in the step S10 of FIG. 14. The autofocusing of the present operation example is thus completed (S33).

In the operation example of FIG. 14 and the operation example of FIG. 18, two imaging operations (the first imaging and the second imaging) are performed to achieve autofocusing. In a modification example of the operation example of FIG. 18, autofocusing can be achieved with one instance of imaging operation. In the present modification example, the ophthalmic apparatus 1, for example, outputs illumination light from only one of the two apertures 21A and 21B of the iris diaphragm 21 to perform imaging. The ophthalmic apparatus 1 then determines the position of the slit light image depicted in the fundus image acquired by this imaging, obtains an estimated value of the refractive power of the subject's eye based on the relative position between the position of the slit light image and a predetermined reference position, determines a focus adjustment condition from the estimated value, and controls the focus adjusting unit 3 based on the focus adjustment condition. The reference position used in the present modification example may be the same as the reference position in the operation example of FIG. 18.

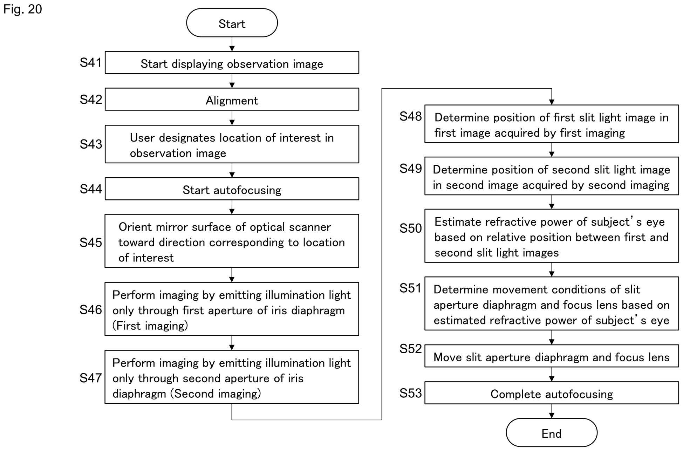

FIG. 20 shows yet another example of the operation for achieving autofocusing performed by the ophthalmic apparatus 1 according to the first embodiment example.

In the aforementioned operation example of FIG. 14 and the aforementioned operation example of FIG. 18, focus adjustment is performed in such a manner as to bring a predetermined location on the fundus Ef of the subject's eye E (e.g., the center of the fundus, the center of the image frame) into focus. In contrast to this, the present operation example of FIG. 20 is capable of performing focus adjustment in a way that a freely selected or determined location on the fundus Ef is brought into focus. This capability allows for the acquisition of an image in which any site of interest on the fundus Ef is clearly depicted. The site of interest may be, for example, any tissue such as the optic nerve head, macula, or blood vessels, or may be a lesion, treatment scar, or the like.

In the present operation example, the ophthalmic apparatus 1 first starts generation and display of an observation image (live moving image, live video, time-series image) of the subject's eye E (S41). The generation of the observation image can be performed by a freely selected or determined method. In some examples, the generation of the observation image may be performed by the use of a means for alignment (e.g., two anterior segment cameras for stereo alignment, or a camera for generating a front image), or may be performed by means of repetitive slit scanning. The display of the observation images is carried out using the user interface 6 (the display device). Based on the observation image obtained in the step S41, the ophthalmic apparatus 1 performs alignment (S42).

In some aspect examples, after the completion of the alignment in the step S42, the user designates a desired location (location of a site of interest, location of interest) in the displayed observation image using the user interface 6 (the input unit) (S43). For instance, the user may touch the desired location in the observation image displayed on a touchscreen. Alternatively, the user may designate the desired location in the observation image displayed on the display device using a pointing device.

In some other aspect examples, the processor 4 may analyze an observation image to determine a location of interest. In still other aspect examples, the user or the processor 4 may designate a location of interest in an image of the fundus Ef acquired in the past examination, and the processor 4 may perform registration between this past image and an observation image to identify the location in the observation image corresponding to the location of interest in the past image. In this case, the identified location in the observation image is used as the location of interest in the step S43. In yet other aspect examples, the user or the processor 4 may designate a location of interest in an observation image generated prior to alignment, and the processor 4 may perform alignment by using the designated location of interest as a reference.

The ophthalmic apparatus 1 starts autofocusing with respect to the location of interest on the fundus Ef designated in the step S43 (S44).

In the autofocusing process of the present operation example, the projection controller 401 first controls the optical scanner 30 to orient the mirror surface thereof toward the direction corresponding to the location of interest on the fundus Ef designated in the step S43 (S45). The orientation of the mirror surface of the optical scanner 30 is maintained in that direction. As evident from the operation for slit scanning (e.g., FIG. 5), there is a predefinable relationship between positions or locations (Y coordinates) in an image acquired by the imaging unit 2 and orientations of the mirror surface of the optical scanner 30. The process of the step S45 may be performed with reference to this relationship.

In the state where the orientation of the mirror surface of the optical scanner 30 is fixed in the direction corresponding to the location of interest on the fundus Ef designated in the step S43, the projection controller 401 controls the imaging unit 2 to perform the first imaging and the second imaging (S46 and S47).

The processes in the steps S48 to S53 of the present operation example may be performed in the same manner as the processes in the steps S6 to S11 of the operation example in FIG. 14, respectively. Note that, some aspect examples may perform the processes in the steps S26 to S33 of the operation example in FIG. 18 in place of the processes in the steps S48 to S53.

The following describes some features and some effects of the ophthalmic apparatus 1 according to the first embodiment example.

The ophthalmic apparatus 1 includes the imaging unit 2, the focus adjusting unit 3, and the processor 4. The imaging unit 2 is configured to perform imaging of the fundus Ef of the subject's eye E with imaging device 50 of the rolling shutter type (or with an imaging device having similar functionality as an imaging device of a rolling shutter type, such as an imaging unit that has a combined configuration of an image sensor of the global shutter type and a slit diaphragm) while moving a projection position of slit light relative to the fundus Ef of the subject's eye E. The focus adjusting unit 3 is configured for performing focus adjustment of the imaging unit 2. The processor 4 is configured to perform slit light projection control, a condition determination process, and focus adjustment control. In the slit light projection control, the processor 4 controls the imaging unit 2 to project the slit light onto the fundus Ef. In the condition determination process, the processor 4 determines a focus adjustment condition based on an output from the imaging device 50 that has detected return light of the slit light projected onto the fundus Ef under the slit light projection control. In the focus adjustment control, the processor 4 controls the focus adjusting unit 3 based on the focus adjustment condition determined by the condition determination process.

According to the ophthalmic apparatus 1 according to the first embodiment example configured as described above, it becomes possible to perform autofocusing by projecting slit light onto the fundus Ef using the imaging unit 2 that conducts slit scanning (which is an imaging modality that is a combination of the operation of moving the projection position of the slit light and the operation of repeating imaging of the fundus Ef of the subject's eye E by the use of the rolling shutter type imaging device 50), and by determining the focus condition based on information obtained by performing imaging of the fundus Ef onto which the slit light is being projected. Therefore, according to the ophthalmic apparatus 1, it becomes possible to perform focus adjustment without having to incorporate a dedicated hardware component for focusing operations, as is provided in conventional ophthalmic apparatuses of similar types. At the very least, according to the ophthalmic apparatus 1, focus adjustment can be performed without providing a complex and large-scale dedicated hardware component for focus as found in conventional ophthalmic apparatuses of similar types. This makes it possible to achieve miniaturization of ophthalmic apparatuses, simplification in the structure of ophthalmic apparatuses, and reduction in manufacturing costs of ophthalmic apparatuses.

Some features that can be adopted in some aspect examples for implementing the ophthalmic apparatus 1 according to the first embodiment example will be described below. It should be noted that all of these features are non-limiting examples. Two or more of these features may be combined, at least in part. In addition, one or more of these features may be combined, at least in part, with other matters or items, for example, with matters or items described in the present disclosure, or with known matters or items.

In some aspect examples, the processor 4 may include the condition determining processor 402 configured to perform the condition determination process. The condition determining processor 402 may be configured to determine the focus adjustment condition based on the position of the slit light image in the image generated by the imaging device 50 that detects the return light of the slit light projected onto the fundus Ef under the slit light projection control.

In some aspect examples, the condition determining processor 402 may further be configured to perform the following processes: a process of generating an intensity profile in an image region that is at least a part of the slit light image in the image generated by the imaging device 50 that detects the return light of the slit light projected onto the fundus Ef under the slit light projection control, wherein the intensity profile is defined along the first direction (the scan direction, the Y direction, the slit width direction) corresponding to the direction in which the projection position of the slit light is moved by the slit scanning; and a process of determining the position of the slit light image based on the intensity profile.

In some aspect examples, the condition determining processor 402 may further be configured to perform the generation of the intensity profile by summing, in the second direction (the X direction, the slit length direction) perpendicular to the first direction, the intensities of a group of pixels constituting the image region that is at least a part of the slit light image in the image generated by the imaging device 50 that detects the return light of the slit light projected onto the fundus Ef under the slit light projection control.

In some aspect examples, the imaging unit 2 may include the illumination optical system 20 (the first optical system) configured to project the slit light onto the fundus Ef of the subject's eye E, and the photographing optical system 40 (the second optical system) configured to guide the return light of the slit light from the fundus Ef to the imaging device 50. Furthermore, the focus adjusting unit 3 may include the first focus adjusting unit configured for adjusting the focal position of the illumination optical system 20, and the second focus adjusting unit configured for adjusting the focal position of the photographing optical system 40. In this case, the condition determining processor 402 may be configured to determine, as the focus adjustment condition, a condition used for controlling the first focus adjusting unit (the first focus adjustment condition) and a condition used for controlling the second focus adjusting unit (the second focus adjustment condition).

In some aspect examples, the illumination optical system 20 may include the light source 10, and the slit aperture diaphragm 22 (slit diaphragm) configured for generating the slit light from light emitted by the light source 10, and may be configured to project the slit light generated by the slit aperture diaphragm 22 onto the fundus Ef of the subject's eye E. Furthermore, the photographing optical system 40 may include the focus lens 47 (focus lens). In addition, the first focus adjusting unit may include the moving mechanism 22M (the first moving mechanism) configured for moving the slit aperture diaphragm 22 in the direction along the optical axis of the illumination optical system 20, and the second focus adjusting unit may include the moving mechanism 47M (the second moving mechanism) configured for moving the focus lens 47 in the direction along the optical axis of the photographing optical system 40. In this case, the condition determining processor 402 may be configured to determine a condition used for controlling the moving mechanism 22M (the first movement control condition) as the first focus adjustment condition, and to determine a condition used for controlling the moving mechanism 47M (the second movement control condition) as the second focus adjustment condition.

In some aspect examples, the first movement control condition used for controlling the moving mechanism 22M may include information indicating a movement direction and a movement distance of the slit aperture diaphragm 22, and the second movement control condition used for controlling the moving mechanism 47M may include information indicating a movement direction and a movement distance of the focus lens 47. The information indicating the movement direction may be any type of information equivalent to the movement direction, such as the content of a control signal corresponding to the movement direction (e.g., the polarity (positive or negative) of a control pulse signal). Likewise, the information indicating the movement distance may be any type of information equivalent to the movement distance, such as the content of a control signal corresponding to the movement distance (e.g., the number pulses of a control pulse signal).

In some aspect examples, the processor 4 (the projection controller 401) may be configured to control the imaging unit 2 in the slit light projection control to emit the slit light projected onto the fundus Ef of the subject's eye E from a position spaced a predetermined distance (height h) from the optical axis of the imaging unit 2. In this case, the condition determining processor 402 may be configured to determine the focus adjustment condition based on the following pieces of information: the position (distance Δ') of the slit light image in the image generated by the imaging device 50 that detects the return light of the slit light projected onto the fundus Ef under the slit light projection control; the focal length (F) of the imaging unit 2; and the height h.

In some aspect examples, the imaging unit 2 may include the optical scanner 30 (optical scanner) configured to deflect the slit light guided to the subject's eye E to move the projection position of the slit light relative to the fundus Ef. Furthermore, the processor 4 may include the projection controller 401 (projection controller) configured to perform the slit light projection control. The projection controller 401 may be configured to control the imaging unit 2 to project slit light onto the fundus Ef in a state where the direction in which the optical scanner 30 deflects the slit light is maintained. In addition, the condition determining processor 402 may include the projection controller 401 configured to perform the slit light projection control. Furthermore, the condition determining processor 402 may be configured to determine the focus adjustment condition based on the position of a slit light image corresponding to the slit light projected onto the fundus Ef in the state where the deflection direction by the optical scanner 30 is fixed.

In some aspect examples, the processor 4 may include the projection controller 401 (projection controller) configured to perform the slit light projection control. The projection controller 401 may be configured to perform the control of the imaging unit to output the first slit light and the second slit light that are projected onto mutually different locations on the fundus Ef of the subject's eye E. The projection of the first slit light and the projection of the second slit light may be performed, for example, in a sequential manner or in a simultaneous manner. Furthermore, the condition determining processor 402 may be configured to determine the focus adjustment condition based on the relative position between the first slit light image corresponding to the first slit light and the second slit light image corresponding to the second slit light.

### < Second embodiment example >

An ophthalmic apparatus according to the second embodiment example will be described. Similar to the ophthalmic apparatus 1 according to the first embodiment example, the ophthalmic apparatus according to the second embodiment example has the ophthalmic imaging function that performs imaging (visualization) of the fundus of a living eye using a slit-scanning modality, and includes the imaging unit 2, the focus adjusting unit 3, the processor 4, the memory 5, and the user interface 6. Any matters or items according to the first embodiment example may be applied to the ophthalmic apparatus according to the second embodiment example. The second embodiment example will be described below with appropriate reference to the matters or items according to the first embodiment example.

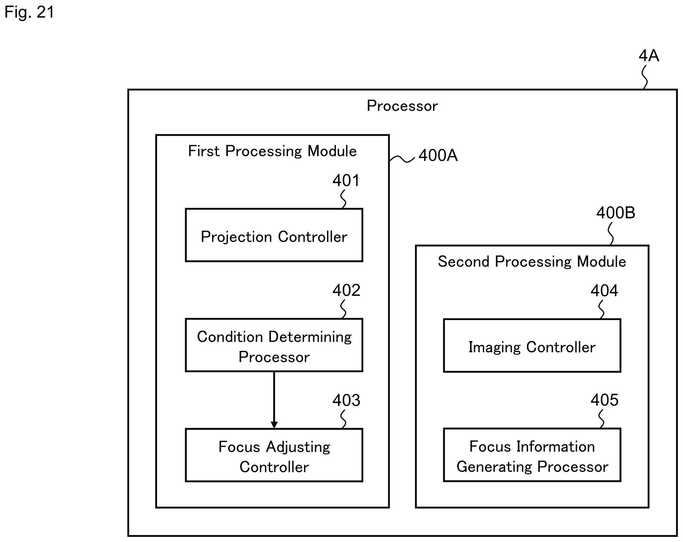

The ophthalmic apparatus according to the second embodiment example includes the processor 4A shown in FIG. 21 in place of the processor 4 shown in FIG. 6. The processor 4A is a non-limiting example. The processor 4A includes the first processing module 400A and the second processing module 400B.

The first processing module 400A is configured to perform processing for focus adjustment (in particular, autofocusing) and includes the projection controller 401, the condition determining processor 402, and the focus adjusting controller 403. The projection controller 401, the condition determining processor 402, and the focus adjusting controller 403 each have the same configuration as the corresponding components in the first embodiment example. For details regarding these components (configuration, function, operation, etc.), refer to the first embodiment example.

The second processing module 400B is configured to perform processing for monitoring the focus state while an observation image (live moving image, live video, time-series image) of the fundus Ef of the subject's eye E is being acquired using slit scanning. The second processing module 400B includes the imaging controller 404 and the focus information generating processor 405.

The imaging controller 404 is configured to perform, in parallel, control (the first imaging control) to cause the imaging unit 2 to acquire an observation image of the fundus Ef and control (the second imaging control) of the imaging unit 2 to detect the focus state of the imaging unit 2. The manner or mode of the parallel performance of the first imaging control and the second imaging control may be freely selected or determined, and may be, for example, any of simultaneous performance, synchronous performance, alternating (interchanging) performance, switching performance, and other modes.

In the first imaging control, the imaging controller 404 is configured to control the imaging unit 2 to acquire a time-series image of the fundus Ef of the subject's eye E, by repeatedly performing a series of imaging of the fundus Ef of the subject's eye E by the use of the imaging device 50 while repeating a series of movement of the projection position of the slit light relative to the fundus Ef of the subject's eye E. The first imaging control, for example, repeatedly performs the slit scanning shown in FIG. 5. As a result, a plurality of images (time-series image) corresponding to the repetition of the slit scanning can be acquired, and an observation image of the fundus Ef can be provided by real-time display of the time-series image on the display device of the user interface 6.

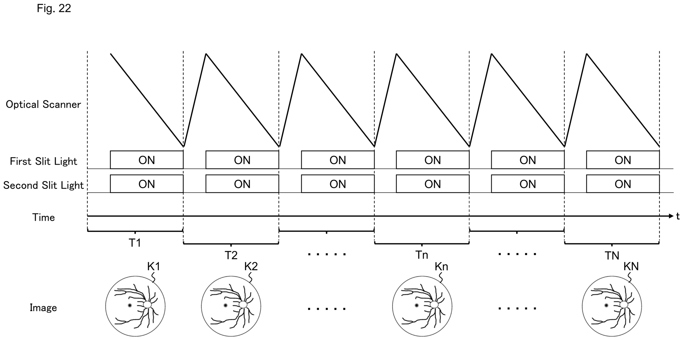

A non-limiting example of the first imaging control will be described with reference to FIG. 22. It should be noted that the aspect example shown in FIG. 22 does not incorporate the second imaging control, which will be described later, and illustrates only the first imaging control. In addition, the aspect example shown in FIG. 22 represents one example of conventional technique for fundus observation by means of slit scanning. The processing according to the second embodiment example will be described subsequently.

The first imaging control illustrated in FIG. 22 includes synchronized and repetitive control of the operation of the optical scanner 30 and the output of slit light (the first slit light and the second slit light). Although not shown in the drawing, the following operations are also performed in synchronization with the control shown in FIG. 22: the operation of repeatedly controlling to read out signals from the imaging device 50 (the image sensor 51) by means of the rolling shutter method; and the operation of repeatedly controlling to display images (the time-series image) sequentially acquired by the imaging device 50 in real time.

In the first imaging control of the present example, the imaging controller 404 varies the orientation of the mirror surface of the optical scanner 30 over a predetermined angular range during the first period T1. In FIG. 22, the uppermost point of the sawtooth pulse (triangular wave pulse) representing the operation of the optical scanner 30 corresponds to the scan start angle, and the lowermost point corresponds to the scan end angle. Here, the scan start angle indicates the initial angle (initial direction) of the orientation of the mirror surface during one instance of slit scan (a single slit scan), and the scan end angle indicates the terminal angle (terminal direction) of the orientation of the mirror surface during one instance of slit scan. That is, the angular range defined by the scan start angle and the scan end angle corresponds to the angular range over which the orientation of the mirror surface changes during one instance of slit scan.

During the first period T1, the imaging controller 404 performs control of the light source 10 to output light (infrared light, near-infrared light) in synchronization with control of the optical scanner 30 to change the orientation of the mirror surface from the scan start angle to the scan end angle. More specifically, the imaging controller 404 performs synchronized control between the optical scanner 30 and the light source 10 in such a manner as to cause the light source 10 to start light emission at the timing when the mirror surface of the optical scanner 30 begins to move from the scan start angle, and to cause the light source 10 to stop the light emission at the timing when the mirror surface of the optical scanner 30 reaches the scan end angle. With this synchronized control, one instance of slit scan using the first slit light and the second slit light is performed (refer to FIG. 5). The one instance of slit scan performed during the first period T1 yields one image K1 corresponding to the first period T1.

Upon completion of the slit scanning during the first period T1, the imaging controller 404 performs preparation for the slit scanning in the second period T2. Specifically, the imaging controller 404 controls the optical scanner 30 in such a manner as to return the mirror surface, which is oriented at the scan end angle at the end of the first period T1, to the scan start angle. Once the mirror surface has been moved to the scan start angle, the imaging controller 404 controls the imaging unit 2 to perform the slit scanning during the second period T2. This control is carried out in the same manner as the slit scanning during the first period T1. As a result of this, one image K2 corresponding to the second period T2 is generated.

By performing such control during each period Tn (n is a positive integer equal to or less than N), N pieces of images K1 to KN respectively corresponding to the N pieces of periods T1 to TN are obtained. The group of the images K1 to KN is a time-series image. As described above, the time-series image is displayed in real time on the display device of the user interface 6. That is, at the timing when the image Kn for each period Tn is generated, the processor 4A displays this image Kn on the display device in place of the previously displayed image K(n-1). Through this process, the sequentially acquired time-series image (the images K1 to KN) is provided to the user as a live video (as an observation image). This concludes the description of the first imaging control.

Next, the second imaging control will now be described. In the second imaging control, the imaging controller 404 controls the imaging unit 2 to perform imaging for detecting a focus state of the imaging unit 2 relative to the fundus Ef of the subject's eye E. The second imaging control performs control of a different mode from that of the first imaging control and represents a novel mode of control that is not implemented in existing ophthalmic apparatuses of similar types. Of course, it is also a novel operation to perform the first imaging control and the second imaging control in combination with each other.

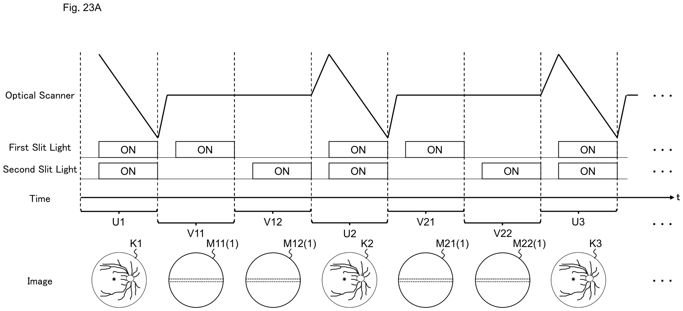

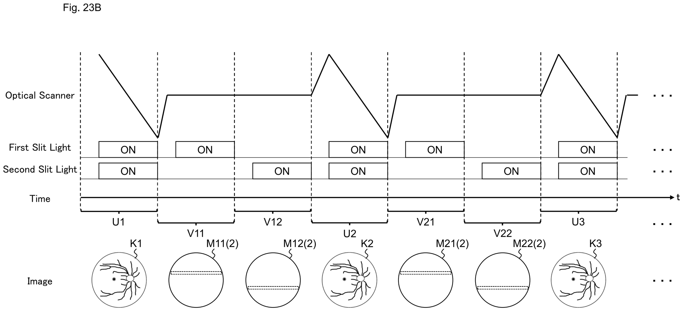

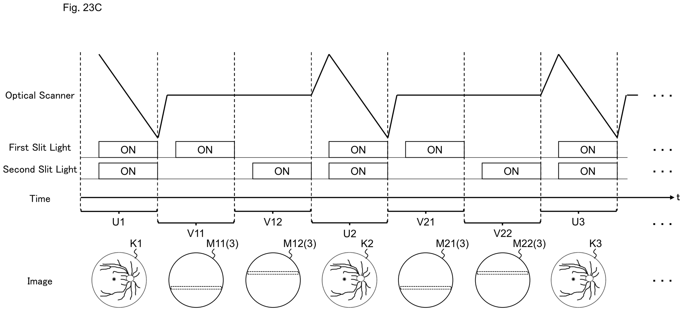

FIG. 23A, FIG. 23B, and FIG. 23C illustrate non-limiting examples of the combination of the first imaging control and the second imaging control. In these examples, one instance of the first imaging control and one instance of the second imaging control are alternately performed. More generally, one or more instances of the first imaging control and one or more instances of the second imaging control may be alternately performed. FIG. 23A shows a case where the imaging unit 2 is in focus with the fundus Ef, FIG. 23B shows a case where the imaging unit 2 is in a back-focused state relative to the fundus Ef, and FIG. 23C shows a case where the imaging unit 2 is in a front-focused state relative to the fundus Ef.

During the period U1, the imaging controller 404 performs one instance of slit scan (i.e., one instance of the first imaging control) as performed during the first period T1 in FIG. 22. This results in the acquisition of the image K1 corresponding to the period U1.

Upon completion of the slit scanning during the period U1, the imaging controller 404 varies the orientation of the mirror surface of the optical scanner 30 from the scan end angle to the neutral position (0-degree position), and then starts the second imaging control. In this second imaging control, the imaging controller 404 causes the imaging unit 2 to perform imaging using the first slit light during the period V11, and causes the imaging unit 2 to perform imaging using the second slit light during the period V12. The imaging using the first slit light and the imaging using the second slit light may be performed by means of the same method as those described in the first embodiment example.

The image M11(1) shown in FIG. 23A, the image M11(2) shown in FIG. 23B, and the image M11(3) shown in FIG. 23C represent three examples of images acquired by performing imaging with the first slit light during the period V11. The images M11(1), M11(2), and the image M11(3) are an image obtained in the case where the imaging unit 2 is in focus, an image obtained in the case where the imaging unit 2 is back-focused, and an image obtained in the case where the imaging unit 2 is front-focused relative to the fundus Ef, respectively. The slit light images depicted in these images are indicated by dashed lines.

Similarly, the image M12(1) shown in FIG. 23A, the image M12(2) shown in FIG. 23B, and the image M12(3) shown in FIG. 23C represent three examples of images acquired by performing imaging with the second slit light during the period V12. The images M12(1), M12(2), and the image M12(3) are an image obtained in the case where the imaging unit 2 is in focus, an image obtained in the case where the imaging unit 2 is back-focused, and an image obtained in the case where the imaging unit 2 is front-focused relative to the fundus Ef, respectively. The slit light images depicted in these images are indicated by dashed lines.

The slit light image in the image M11(1) of FIG. 23A is depicted substantially at the central position in the Y direction, and the slit light image in the image M12(1) is also depicted substantially at the central position in the Y direction. As described in the first embodiment example, the positional relationship between the two slit light images in the two images M11(1) and M12(1) of FIG. 23A indicates that the imaging unit 2 is substantially in focus with the fundus Ef.

In contrast, the positional relationship between the two slit light images in the two images M11(2) and M12(2) of FIG. 23B indicates that the imaging unit 2 is not in focus with the fundus Ef, or more specifically, that the imaging unit 2 is back-focused relative to the fundus Ef. Furthermore, the positional relationship between the two slit light images in the two images M11(3) and M12(3) of FIG. 23C indicates that the imaging unit 2 is not in focus with the fundus Ef, or more specifically, that the imaging unit 2 is in a front-focused state relative to the fundus Ef.

The focus information generating processor 405 may generate information (focus information) indicating the focus state of the imaging unit 2 during the periods V11 and V12, based on the two images acquired during the periods V11 and V12. The focus information may include any of the following pieces of information, for example: information indicating whether or not the fundus Ef is in focus of the imaging unit 2; information indicating the direction of defocus of the imaging unit 2 (i.e., information indicating the focus state of the imaging unit 2, that is, whether the imaging unit 2 is back-focused or front-focused relative to the fundus Ef); and information indicating the amount of defocus of the imaging unit 2.

The processor 4A may output display information based on the focus information generated, to the display device of the user interface 6.

Upon completion of the slit scanning during the period V12, the imaging controller 404 moves the orientation of the mirror surface of the optical scanner 30 from the neutral position (0-degree position) to the scan start angle, and then starts the second instance of the first imaging control (slit scanning). As a result, one image K2 corresponding to the period U2 is generated.

Upon completion of the slit scanning during the period U2, the imaging controller 404 moves the orientation of the mirror surface of the optical scanner 30 from the scan end angle to the neutral position (0-degree position), and then starts the second instance of the second imaging control. The second imaging control yields two images corresponding to the two periods V21 and V22. For example, the two images M21(1) and M22(1) in FIG. 23A, the two images M21(2) and M22(2) in FIG. 23B, or the two images M21(3) and M22(3) in FIG. 23C may be obtained.

The focus information generating processor 405 may generate information (focus information) indicating the focus state of the imaging unit 2 during the periods V21 and V22, based on the two images acquired during the periods V21 and V22. The processor 4A may output display information based on the focus information generated, to the display device of the user interface 6.

In the manner described above, the ophthalmic apparatus of the second embodiment example alternately performs the first imaging control and the second imaging control. This alternate control enables monitoring of the focus state of the imaging unit 2 relative to the fundus Ef in parallel with generation of an observation image (time-series image K1, K2, K3, ...) of the fundus Ef using slit scanning.

Some features and some effects of the ophthalmic apparatus according to the second embodiment example are described below.

The imaging unit 2 of the ophthalmic apparatus of the second embodiment example may be configured to acquire a time-series image of the fundus Ef of the subject's eye E, by repeatedly performing a series of imaging of the fundus Ef of the subject's eye E using the imaging device 50 while repeating a series of movement of the projection position of the slit light relative to the fundus Ef of the subject's eye E. In other words, the imaging unit 2 may be configured to generate a time-series image (observation image) by repeatedly performing slit scanning. In addition to the configuration for performing the slit light projection control, the condition determination process, and the focus adjustment control that are performed by the ophthalmic apparatus 1 according to the first embodiment, the processor 4A of the second embodiment example is configured to perform the first imaging control, the second imaging control, and the focus information generation process in parallel. In the first imaging control, the processor 4A performs control for causing the imaging unit 2 to acquire the time-series image of the fundus Ef. In the second imaging control, the processor 4A performs control to cause the imaging unit 2 to perform imaging for detecting the focus state of the imaging unit 2 relative to the fundus Ef. In the focus information generation process, the processor 4A performs generation of focus information that indicates the focus state of the imaging unit 2 based on an image acquired by the imaging performed under the second imaging control.

The ophthalmic apparatus according to the second embodiment example configured in this manner makes it possible to achieve miniaturization of ophthalmic apparatuses, simplification in the structure of ophthalmic apparatuses, and reduction in manufacturing costs of ophthalmic apparatuses, like the first embodiment example. In addition, the ophthalmic apparatus according to the second embodiment example is capable of monitoring the focus state of the imaging unit in parallel with the generation of the observation image using slit scanning.

In some aspect examples, the processor 4A may be configured to alternately perform one or more instances of the first imaging control and one or more instance of the second imaging control.

In some aspect examples, the processor 4A may be configured to further perform display control in parallel with the first imaging control, the second imaging control, and the focus information generation process. In the display control, the processor 4A outputs display information based on the focus information, to the display device of the user interface 6. This configuration allows for miniaturization of ophthalmic apparatuses, simplification in the structure of ophthalmic apparatuses, and reduction in manufacturing costs of ophthalmic apparatuses. Moreover, it enables real-time monitoring of the focus state of the imaging unit and real-time provision of the focus state of the imaging unit to the user, in parallel with the observation image generation by means of slit scanning. Some non-limiting examples of the display control will be described in the fifth embodiment.

### < Third embodiment example >

The ophthalmic apparatus according to the third embodiment example will now be described. Similar to the ophthalmic apparatus 1 according to the first embodiment example and the ophthalmic apparatus according to the second embodiment example, the ophthalmic apparatus according to the third embodiment example has the ophthalmic imaging function that performs imaging (visualization) of the fundus of a living eye using a slit-scanning modality, and includes the imaging unit 2, the focus adjusting unit 3, the processor 4, the memory 5, and the user interface 6. Any matters or items according to the first embodiment example and any matters or items according to the second embodiment example may be applied to the ophthalmic apparatus according to the third embodiment example. The third embodiment example will be described below with appropriate reference to the matters or items according to the first embodiment example and the matters or items according to the second embodiment example.

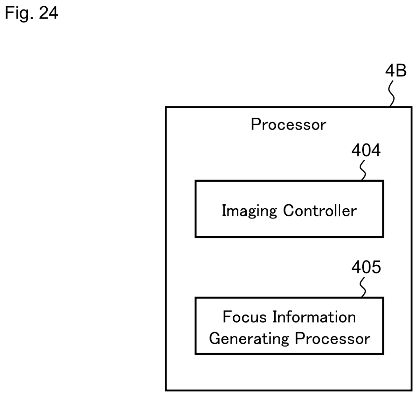

The ophthalmic apparatus according to the third embodiment example includes the processor 4B as shown in FIG. 24 in place of the processor 4 shown in FIG. 6. The processor 4B is a non-limiting example. Similar to the second processing module 400B of the processor 4A of the second embodiment example, the processor 4B includes the imaging controller 404 and the focus information generating processor 405. In other words, the ophthalmic apparatus according to the third embodiment example has a configuration equivalent to that of the ophthalmic apparatus of the second embodiment example with the first processing module 400A removed from the processor 4A.

With this configuration, the ophthalmic apparatus according to the third embodiment example is capable of monitoring the focus state of the imaging unit in parallel with observation image generation using slit scanning, similar to the second embodiment example. A non-limiting example of the display control for monitoring the focus state will be described in the fifth embodiment.

### < Fourth embodiment example >

Similar to the ophthalmic apparatus according to the first to third embodiment examples, the ophthalmic apparatus according to the fourth embodiment example has the ophthalmic imaging function that performs imaging (visualization) of the fundus of a living eye using a slit-scanning modality, and includes the imaging unit 2, the focus adjusting unit 3, the processor 4, the memory 5, and the user interface 6. Any matters or items according to the first embodiment example, any matters or items according to the second embodiment example, and any matters or items according to the third embodiment example may be applied to the ophthalmic apparatus according to the fourth embodiment example. The fourth embodiment example will be described below with appropriate reference to the matters or items according to the first to third embodiment examples.

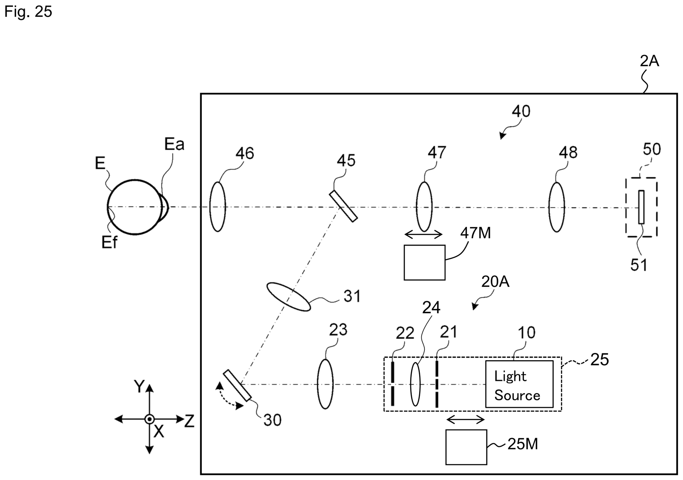

The ophthalmic apparatus according to the fourth embodiment example includes the imaging unit 2A as illustrated in FIG. 25 in place of the imaging unit 2 shown in FIG. 2. The imaging unit 2Ais a non-limiting example. The imaging unit 2Ais configured to be able to perform slit scanning, by performing imaging with an image sensor (imaging device) of a rolling shutter type while moving a projection position of slit light relative to the fundus Ef of the subject's eye E. In place of the image sensor of the rolling shutter type, an imaging unit (imaging device) that is a combination of an image sensor of a global shutter type and a slit diaphragm may be employed.

The imaging unit 2A of the present embodiment example includes the light source 10, the illumination optical system 20A, the optical scanner 30, the photographing optical system 40, and the imaging device 50. The light source 10, the optical scanner 30, the photographing optical system 40, and the imaging device 50 may respectively be the same as the corresponding components in the imaging unit 2 of the first embodiment example. The light source 10 and/or the optical scanner 30 may be considered as components of the illumination optical system 20A, and the imaging device 50 may be considered as a component of the photographing optical system 40.

The illumination optical system 20A of the present embodiment example is provided in place of the illumination optical system 20 of the first embodiment example. In the example shown in FIG. 25, the illumination optical system 20A includes the iris diaphragm 21, the slit aperture diaphragm 22, and the relay lens 23 that have similar configurations to those in the first embodiment example, and further includes the lens 24 disposed between the iris diaphragm 21 and the slit aperture diaphragm 22.

Furthermore, the imaging unit 2A of the present embodiment example includes the moving mechanism 25M. The moving mechanism 25M is configured to move the movable unit 25 that includes the light source 10, the iris diaphragm 21, the lens 24, and the slit aperture diaphragm 22, in the direction along the optical axis of the illumination optical system 20. The moving mechanism 25M operates under the control of the processor 4. The processor 4 may be configured to control the moving mechanism 25M depending on the condition of the subject's eye E, such as refractive power (refractive index, diopter), fundus shape, or other conditions.

The processor 4 of the present embodiment example includes the projection controller 401, the condition determining processor 402, and the focus adjusting controller 403, similar to the processor 4 of the first embodiment (see FIG. 6). The condition determining processor 402 of the present embodiment example performs the condition determination process similar to that of the first embodiment example. More specifically, the condition determining processor 402 is configured to perform a computational process to determine a focus adjustment condition based on an output from the imaging device 50 that has detected return light of the slit light projected onto the fundus Ef under the slit light projection control performed by the projection controller 401. The condition determination process of the present embodiment example may be performed in the same manner as in the first embodiment example.

In the present embodiment example, the illumination optical system 20A (the first optical system) includes the light source 10 (light source), the iris diaphragm 21 (iris diaphragm) that is disposed at a position substantially optically conjugate with the iris of the subject's eye E and that has an aperture through which light emitted by the light source 10 passes, the lens 24 (lens) configured to refract the light that has passed through the aperture of the iris diaphragm 21, and the slit aperture diaphragm 22 (slit diaphragm) configured for generating slit light from the light refracted by the lens 24. With these components, the illumination optical system 20A is configured to project the slit light generated by the slit aperture diaphragm 22 onto the fundus Ef of the subject's eye E. Furthermore, the photographing optical system 40 (the second optical system) includes the focus lens 47 (focus lens). In addition, the first focus adjusting unit configured for adjusting the focal position of the illumination optical system 20A includes the moving mechanism 25M (the third moving mechanism) configured for moving the movable unit 25 in the direction along the optical axis of the illumination optical system 20A. In other words, the first focus adjusting unit includes the moving mechanism 25M configured for integrally moving the light source 10, the iris diaphragm 21, the lens 24, and the slit aperture diaphragm 22 in the direction along the optical axis of the illumination optical system 20A. In addition, the second focus adjusting unit configured for adjusting the focal position of the photographing optical system 40 includes the moving mechanism 47M (the fourth moving mechanism) configured for moving the focus lens 47 in the direction along the optical axis of the photographing optical system 40. Moreover, the condition determining processor 402 is configured to determine a condition (the third movement control condition, the first focus adjustment condition) used for controlling the moving mechanism 25M, and determine a condition (the fourth movement control condition, the second focus adjustment condition) used for controlling the moving mechanism 47M.

The ophthalmic apparatus according to the fourth embodiment example having the above-described configurations can achieve miniaturization of ophthalmic apparatuses, simplification in the structure of ophthalmic apparatuses, and reduction in the manufacturing cost of ophthalmic apparatuses, like the first embodiment example. Additionally, the ophthalmic apparatus according to the fourth embodiment example can provide an alternative aspect example of the ophthalmic apparatus according to the first embodiment example.

### < Fifth embodiment example >

Similar to the ophthalmic apparatus according to the first to fourth embodiment examples, the ophthalmic apparatus according to the fifth embodiment example has the ophthalmic imaging function that performs imaging (visualization) of the fundus of a living eye using a slit-scanning modality, and includes the imaging unit 2, the focus adjusting unit 3, the processor 4, the memory 5, and the user interface 6. Any matters or items according to the first embodiment example, any matters or items according to the second embodiment example, any matters or items according to the third embodiment example, and any matters or items according to the fourth embodiment example may be applied to the ophthalmic apparatus according to the fifth embodiment example. The fifth embodiment example will be described below with appropriate reference to the matters or items according to the first to fourth embodiment examples.

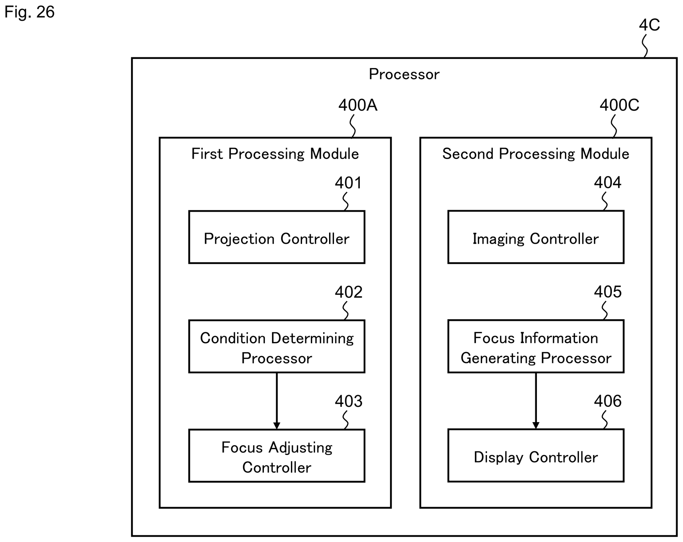

The ophthalmic apparatus according to the fifth embodiment example has the processor 4C as illustrated in FIG. 26, in place of the processor 4 shown in FIG. 6. The processor 4C is a non-limiting example. The processor 4C includes the first processing module 400A and the second processing module 400C.

The first processing module 400A is configured to perform processing for focus adjustment (in particular, autofocusing), and includes the projection controller 401, the condition determining processor 402, and the focus adjusting controller 403, like the first processing module 400A of the second embodiment example (FIG. 21).

The second processing module 400C is configured to perform information display for monitoring the focus state during acquisition of an observation image (live moving image, live video, time-series image) of the fundus Ef of the subject's eye E by means of slit scanning. The second processing module 400C includes the display controller 406, in addition to the imaging controller 404 and the focus information generating processor 405, which are similar to those in the second processing module 400B of the second embodiment example (FIG. 21).

The display controller 406 performs control to output display information based on the focus information (i.e., information indicating the focus state of the imaging unit 2) generated by the focus information generating processor 405, to the display device of the user interface 6. The display information may be any type of information generated based at least on the focus information, and may include, for example, any of the following pieces information: information indicating whether or not the fundus Ef is in focus of the imaging unit 2; information indicating the direction of defocus of the imaging unit 2 (i.e., information indicating the focus state of the imaging unit 2, that is, whether the imaging unit 2 is back-focused or front-focused relative to the fundus Ef); and information indicating the amount of defocus of the imaging unit 2. The form or aspect of the display information may be freely selected or determined, and may be any type of geometric representation or any type of visual representation, such as a medical image, a schema, an illustration, a computer graphics image, a character string, a graph, a diagram, or other types of representation.

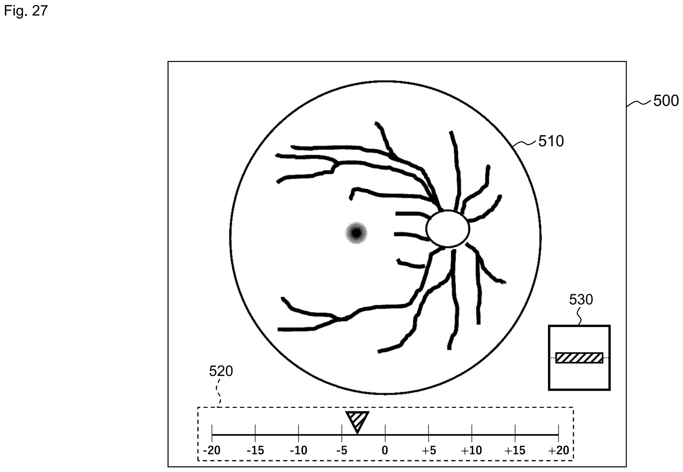

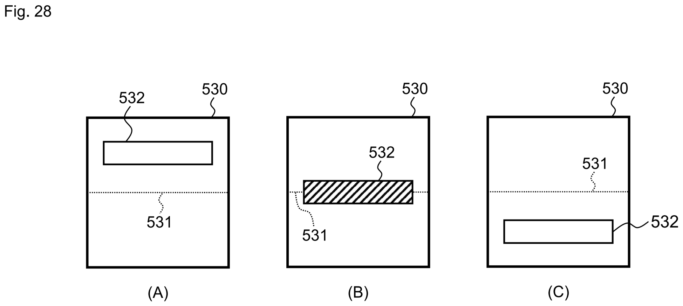

Non-limiting examples of the display information are shown in FIG. 27 and FIG. 28. The display screen 500 shown in FIG. 27 is displayed on the display device of the user interface 6 by the display controller 406. The display screen 500 is provided with the image display section 510 on which an image of the fundus Ef generated by the imaging unit 2 is displayed. The image displayed on the image display section 510 is, for example, an observation image acquired by the imaging unit 2 under the first imaging control performed by the imaging controller 404. The first imaging control is a control operation for causing the imaging unit 2 to acquire the observation image of the fundus Ef; for details, refer to the second embodiment example.

Furthermore, the display screen 500 includes the focus adjusting operation unit 520 and the focus state indicator 530. The focus adjusting operation unit 520 is a graphical user interface (GUI) used for performing focus adjustment operations. The focus state indicator 530 is a graphical user interface that provides representations or visualizations of the direction and the amount of defocus of the imaging unit 2.

The focus adjusting operation unit 520 includes a scale indicating the range of diopter values of the imaging unit 2 (the illumination optical system 20, the photographing optical system 40) and a pointer indicating a current diopter value on the scale.

The scale in the non-limiting example shown in FIG. 27 is a number line indicating the range from minus 20 diopters to plus 20 diopters, with numerical values indicated at intervals of 5 diopters.

The pointer in the non-limiting example shown in FIG. 27 is an inverted triangular (wedge-shaped) widget, with its downward-pointing vertex indicating a point on the scale. The point indicated by the pointer corresponds to the current diopter value of the imaging unit 2.

The user can move the pointer along the scale, for example, by using a pointing device or by touch operations. When the user moves the pointer, the processor 4C (for example, the focus adjusting controller 403 or the imaging controller 404) performs control of the focus adjusting unit 3 (for example, the moving mechanism 22M or the moving mechanism 25M, and the moving mechanism 47M) based on the diopter value (target value) indicated by the moved pointer, thereby changing the diopter value of the imaging unit 2 to the target value.

The form or aspect of the focus adjustment operation unit (the scale, the pointer, etc.) is not limited to the above example, and may be freely selected or determined. For example, in addition to the scale and the pointer shown in FIG. 27, information indicating the direction in which (and information indicating the amount by which) the pointer should be moved to bring the fundus Ef into focus of the imaging unit 2 (referred to as movement assistance information) may be presented. The movement assistance information may be generated, for example, based on the focus information generated by the focus information generating processor 405. As described above, the focus information is generated based on the image acquired by the imaging unit 2 under the second imaging control performed by the imaging controller 404. Here, the second imaging control is a control operation for detecting the focus state of the imaging unit 2; for details, refer to the second embodiment example.

In another example, the focus adjustment operation unit may include a window-shaped widget in which a current diopter value of the imaging unit 2 is presented, and a widget that is operated to change the diopter value presented in the window-shaped widget.

The focus state indicator 530 presents the direction and the amount of defocus of the imaging unit 2. The display controller 406 performs display control of the focus state indicator 530 based on the focus information generated by the focus information generating processor 405 from the image acquired by the imaging unit 2 under the second imaging control performed by the imaging controller 404.

As shown in FIG. 28, the focus state indicator 530 includes the reference line 531 provided at the central position in the vertical direction, and the moving widget 532 whose display position changes in the vertical direction. The reference line 531 indicates the vertical position corresponding to the state in which the imaging unit 2 is in focus. Positions above the reference line 531 correspond to "front-focused" states while positions below correspond to "back-focused" states. The distance with reference to the reference line 531 represents the amount of defocus. The greater the distance from the reference line 531, the greater the amount of defocus.

The display controller 406 performs evaluation of the focus of the imaging unit 2 based on the focus information generated by the focus information generating processor 405, and determines the display position of the moving widget 532 based on the result of the evaluation. The content of the focus evaluation of the imaging unit 2 may be freely selected or determined, and may include, for example, determination or judgement of whether or not the imaging unit 2 is in focus with the fundus Ef, determination of the direction of defocus relative to the fundus Ef (front-focused or back-focused), estimation of the amount of defocus relative to the fundus Ef, or other processes.

In the case where it is determined that the imaging unit 2 is in focus with the fundus Ef, the display controller 406 displays the moving widget 532 on the reference line 531 (see FIG. 28(B)).

In the case where it is determined that the imaging unit 2 is front-focused by an amount corresponding to the first diopter value relative to the fundus Ef, the display controller 406 displays the moving widget 532 at a position above the reference line 531 and at a distance from the reference line 531 by a distance corresponding to the first diopter value (see FIG. 28(A)).

In the case where it is determined that the imaging unit 2 is back-focused by an amount corresponding to the second diopter value relative to the fundus Ef, the display controller 406 displays the moving widget 532 at a position below the reference line 531 and at a distance from the reference line 531 by a distance corresponding to the second diopter value (see FIG. 28(C)).

As shown in FIG. 28, the aspect of the moving widget 532 can be changed depending on whether or not the imaging unit 2 is in focus with the fundus Ef. For example, the display controller 406 may be configured to display the moving widget 532 in the first color (e.g., blue) when the imaging unit 2 is in focus with the fundus Ef, and to display the moving widget 532 in the second color (e.g., red) when the imaging unit 2 is out of focus with the fundus Ef. In another example, the display controller 406 may be configured to display the moving widget 532 in a color corresponding to the direction of defocus (front-focused or back-focused) of the imaging unit 2. In yet other aspect examples, the display controller 406 may be configured to display the moving widget 532 in a color corresponding to the magnitude of the amount of defocus of the imaging unit 2. The display mode or aspect of the moving widget 532 that is changed depending on the focus state of the imaging unit 2, is not limited to display color, and may be in any aspect, such as shape, brightness, pattern, blinking pattern, or other aspects. In addition, the display controller 406 may be configured to display information (e.g., character string information, image information, etc.) corresponding to the focus state of the imaging unit 2 separately from the moving widget 532. By employing a configuration in which the display mode or aspect of the focus state indicator 530 (e.g., the moving widget 532) is changed depending on the focus state of the imaging unit 2, the user can easily grasp the focus state of the imaging unit 2.

The display controller 406 may be configured to synchronize the display mode or aspect of the moving widget 532 with the display mode or aspect of the focus adjusting operation unit 520. For example, as shown in FIG. 27, the display color of the moving widget 532 and the display color of the pointer of the focus adjusting operation unit 520 may be matched with each other. Note that FIG. 27 indicates the case in which the fundus Ef is in focus when the focus state of the imaging unit 2 is minus 3 diopters (-3 D). The display mode to be synchronized is not limited to display color, and may be in any aspect, such as shape, brightness, pattern, blinking pattern, or other aspects. The synchronization of the display modes between two or more objects is not limited to displaying these objects in the same aspect.

According to an ophthalmic apparatus that can display the display screen 500 described above, the user can grasp the focus state of the imaging unit 2 in real time by referring to the focus state indicator 530 while observing the fundus Ef by referring to the image displayed on the image display section 510. In addition, the user can perform focus adjustment of the imaging unit 2 using the focus adjusting operation unit 520.

### < Other embodiment examples >

While several non-limiting aspect examples of the ophthalmic apparatus according to the embodiment examples have been described above, categories of embodiment examples according to the present disclosure are not limited to ophthalmic apparatuses. Examples of the categories of embodiment examples other than ophthalmic apparatuses include a method for controlling an ophthalmic apparatus, a method for imaging an eye fundus, a program, a recording medium, and other categories. It will be understood by those skilled in the art that the embodiment examples in these categories can be implemented by the aforementioned embodiment examples of the ophthalmic apparatus.

The embodiment examples in these categories, like the embodiment examples of the ophthalmic apparatus, can also achieve the following effects: focus adjustment can be performed without providing a dedicated hardware component for focus as found in existing ophthalmic apparatuses of similar types (i.e., ophthalmic apparatuses having the ophthalmic imaging function that performs imaging or visualization of the fundus of a living eye using a slit-scanning modality); focus adjustment can be performed without providing a complex and large-scale dedicated hardware component for focus as found in conventional ophthalmic apparatuses of similar types; and the focus state of the imaging unit can be monitored in parallel with generation of an observation image using slit scanning.

Described below are several non-limiting embodiment examples of these categories. Note that it is possible to combine any matters or items described regarding ophthalmic apparatuses in the present disclosure, as well as any known techniques or technologies, with any of the embodiment examples according to these categories.

A method for controlling an ophthalmic apparatus according to some embodiment examples provides a novel method for controlling an ophthalmic apparatus including an imaging unit, a focus adjusting unit, a processor, and a memory. Here, the imaging unit is configured to perform imaging with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of a subject's eye. The focus adjusting unit is configured for performing focus adjustment of the imaging unit. The method according to the present embodiment example causes the processor to perform the first control step, a condition determination step, and the second control step. In the first control step, the processor performs control of the imaging unit to project slit light onto the fundus of the subject's eye. In the condition determination step, the processor determines a focus adjustment condition based on an output from the imaging device that has detected return light of the slit light projected onto the fundus of the subject's eye by the first control step. In the second control step, the processor performs control of the focus adjusting unit based on the focus adjustment condition determined by the condition determination step.

A method for controlling an ophthalmic apparatus according to some other embodiment examples provides a novel method for controlling an ophthalmic apparatus including an imaging unit, a processor, and a memory. Here, the imaging unit is configured to perform imaging with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of a subject's eye. The method according to the present embodiment example causes the processor to perform, in parallel, the first imaging control step, the second imaging control step, a focus information generation step, and a display control step. In the first imaging control step, the processor performs control of the imaging unit to acquire a time-series image of the fundus of the subject's eye, by repeatedly performing a series of imaging of the fundus of the subject's eye using the imaging device while repeating a series of movement of the projection position of the slit light relative to the fundus of the subject's eye. In the second imaging control step, the processor causes the imaging unit to perform imaging for detecting a focus state of the imaging unit relative to the fundus of the subject's eye. In the focus information generation step, the processor generates focus information indicating the focus state based on an image acquired by the imaging performed by the second imaging control step. In the display control step, the processor causes a display device to output display information based on the focus information generated by the focus information generation step.

A method for imaging a fundus according to some embodiment examples provides a novel method for performing imaging of the fundus of a subject's eye with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of the subject's eye. The present method includes the following steps: a step of projecting slit light onto the fundus of the subject's eye and detecting return light of the slit light projected onto the fundus by the imaging device; a step of determining a focus adjustment condition based on an output from the imaging device that has detected the return light; a step of performing focus adjustment based on the focus adjustment condition; and a step of performing imaging of the fundus of the subject's eye with the imaging device while moving a projection position of slit light relative to the fundus of the subject's eye after the focus adjustment has been performed.

A method for imaging a fundus according to some other embodiment examples provides a novel method for performing imaging of the fundus of a subject's eye with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of the subject's eye. The present method includes the following steps that are performed in parallel: a step of acquiring a time-series image of the fundus of the subject's eye, by repeatedly performing a series of imaging of the fundus of the subject's eye using the imaging device while repeating a series of movement of the projection position of the slit light relative to the fundus of the subject's eye; a step of acquiring an image by performing imaging for detecting a focus state relative to the fundus of the subject's eye; a step of generating focus information indicating the focus state based on an image acquired by the imaging performed for detecting the focus state; and a step of outputting display information based on the focus information.

A program according to some embodiment examples is a novel computer program executed by an ophthalmic apparatus. This ophthalmic apparatus includes an imaging unit, a focus adjusting unit, a processor, and a memory. The imaging unit is configured to perform imaging with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of a subject's eye. The focus adjusting unit is configured for performing focus adjustment of the imaging unit. The program according to the present embodiment example is configured to cause the processor of the ophthalmic apparatus to perform the first control step, a condition determination step, and the second control step. In the first control step, the processor performs control of the imaging unit to project slit light onto the fundus of the subject's eye under the program according to the present embodiment example. In the condition determination step, the processor determines, under the program according to the present embodiment example, a focus adjustment condition based on an output from the imaging device that has detected return light of the slit light projected onto the fundus of the subject's eye by the first control step. In the second control step, the processor performs, under the program according to the present embodiment example, control of the focus adjusting unit based on the focus adjustment condition determined by the condition determination step.

A program according to some other embodiment examples is a novel computer program executed by an ophthalmic apparatus. This ophthalmic apparatus includes an imaging unit, a processor, and a memory. The imaging unit is configured to perform imaging with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of a subject's eye. The program according to the present embodiment example is configured to cause the processor of the ophthalmic apparatus to perform, in parallel, the first imaging control step, the second imaging control step, a focus information generation step, and a display control step. In the first imaging control step, the processor performs control of the imaging unit, under the program according to the present embodiment example, to acquire a time-series image of the fundus of the subject's eye, by repeatedly performing a series of imaging of the fundus of the subject's eye using the imaging device while repeating a series of movement of the projection position of the slit light relative to the fundus of the subject's eye. In the second imaging control step, the processor causes the imaging unit to perform imaging for detecting a focus state of the imaging unit relative to the fundus of the subject's eye under the program according to the present embodiment example. In the focus information generation step, the processor generates focus information indicating the focus state based on an image acquired by the imaging performed by the second imaging control step under the program according to the present embodiment example. In the display control step, the processor causes a display device to output display information based on the focus information generated by the focus information generation step under the program according to the present embodiment example.

A program according to still other embodiment examples is a novel computer program configured to cause a computer including a processor and a memory, to execute processing for performing imaging of the fundus of a subject's eye with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of the subject's eye. The program according to the present embodiment example is configured to cause the processor of the computer to perform the following steps: a step of executing control to project slit light onto the fundus of the subject's eye and detect return light of the slit light projected onto the fundus by the imaging device; a step of determining a focus adjustment condition based on an output from the imaging device that has detected the return light; a step of performing focus adjustment based on the focus adjustment condition; and a step of executing control to perform imaging with the imaging device while moving a projection position of slit light relative to the fundus of the subject's eye after the focus adjustment has been performed.

A program according to still other embodiment examples is a novel computer program configured to cause a computer including a processor and a memory to execute processing for performing imaging of the fundus of a subject's eye with a rolling shutter type imaging device (or an imaging device having similar functionality as an imaging device of a rolling shutter type, such as an imaging unit combining a global shutter type image sensor and a slit diaphragm) while moving a projection position of slit light relative to the fundus of the subject's eye. The program according to the present embodiment example is configured to cause the processor of the computer to perform the following steps in parallel: a step of acquiring a time-series image of the fundus of the subject's eye, by repeatedly performing a series of imaging of the fundus of the subject's eye using the imaging device while repeating a series of movement of the projection position of the slit light relative to the fundus of the subject's eye; a step of acquiring an image by performing imaging for detecting a focus state relative to the fundus of the subject's eye; a step of generating focus information indicating the focus state based on an image acquired by the imaging performed for detecting the focus state; and a step of outputting display information based on the focus information.

The recording medium according to some embodiment examples is a computer-readable non-transitory recording medium storing the program according to any of the embodiment examples. The computer-readable non-transitory recording medium that can be used as a recording medium according to the embodiment example may be a recording medium in any form. Examples of the recording medium include a magnetic disk, an optical disk, a magneto-optical disk, a semiconductor memory, and any other kinds of recording media.

The present disclosure presents several embodiment examples and several aspect examples thereof. These embodiment examples and aspect examples are merely examples of the present invention, and therefore any modifications (e.g., omissions, substitutions, additions, or the like) within the scope of the present invention can be applied to the embodiment examples and aspect examples presented in the present disclosure.

### [EXPLANATION OF REFERENCE CHARACTERS]

- 1: Ophthalmic apparatus
- 2: Imaging unit
- 3: Focus adjusting unit
- 4, 4A, 4B, 4C: Processor
- 5: Memory
- 6: User interface
- 10: Light source
- 20: Illumination optical system
- 21: Iris diaphragm
- 22: Slit aperture diaphragm
- 22M: Moving mechanism
- 30: Optical scanner
- 47: Focus lens
- 47M: Moving mechanism
- 50: Imaging device
- 401: Projection controller
- 402: Condition determining processor
- 403: Focus adjusting controller
- 404: Imaging controller
- 405: Focus information generating processor
- 406: Display controller
- 500: Display screen
- 520: Focus adjusting operation unit
- 530: Focus state indicator

## Claims

1. An ophthalmic apparatus comprising:
an imaging unit configured to perform imaging with an imaging device while moving a projection position of slit light relative to a fundus of a subject's eye;
a focus adjusting unit configured for performing focus adjustment of the imaging unit; and
a processor,
wherein the processor is configured to perform
slit light projection control to control the imaging unit to project slit light onto the fundus,
a condition determination process to determine a focus adjustment condition based on an output from the imaging device that has detected return light of the slit light projected under the slit light projection control, and
focus adjustment control to control the focus adjusting unit based on the focus adjustment condition determined by the condition determination process.

2. The ophthalmic apparatus of claim 1, wherein the processor includes a condition determining processor configured to perform the condition determination process, wherein the condition determining processor is configured to determine the focus adjustment condition based on a position of a slit light image in an image generated by the imaging device.

3. The ophthalmic apparatus of claim 2, wherein the condition determining processor is configured to determine the position of the slit light image based on an intensity profile in an image region that is at least a part of the slit light image, the intensity profile being determined along a first direction corresponding to a movement direction of the projection position of the slit light by the imaging unit.

4. The ophthalmic apparatus of claim 3, wherein the condition determining processor is configured to generate the intensity profile by summing intensities of a group of pixels constituting the image region in a second direction perpendicular to the first direction.

5. The ophthalmic apparatus of claim 2, wherein
the imaging unit includes
a first optical system configured to project the slit light onto the fundus of the subject's eye, and
a second optical system configured to guide return light of the slit light from the fundus of the subject's eye to the imaging device,
the focus adjusting unit includes
a first focus adjusting unit configured for adjusting a focal position of the first optical system, and
a second focus adjusting unit configured for adjusting a focal position of the second optical system, and
the condition determining processor is configured to determine a first focus adjustment condition used for controlling the first focus adjusting unit and a second focus adjustment condition used for controlling the second focus adjusting unit, as the focus adjustment condition.

6. The ophthalmic apparatus of claim 5, wherein
the first optical system includes
a light source, and
a slit diaphragm configured for generating slit light from light emitted by the light source, and
the first optical system is configured to project the slit light generated by the slit diaphragm onto the fundus,
the second optical system includes a focus lens,
the first focus adjusting unit includes a first moving mechanism configured for moving the slit diaphragm in a direction along an optical axis of the first optical system,
the second focus adjusting unit includes a second moving mechanism configured for moving the focus lens in a direction along an optical axis of the second optical system, and
the condition determining processor is configured to determine a first movement control condition used for controlling the first moving mechanism as the first focus adjustment condition and determine a second movement control condition used for controlling the second moving mechanism as the second focus adjustment condition.

7. The ophthalmic apparatus of claim 6, wherein
the first movement control condition includes information indicating a movement direction and a movement distance of the slit diaphragm, and
the second movement control condition includes information indicating a movement direction and a movement distance of the focus lens.

8. The ophthalmic apparatus of claim 5, wherein
the first optical system includes
a light source,
an iris diaphragm that is disposed at a position substantially optically conjugate with an iris of the subject's eye and has an aperture through which light emitted by the light source passes,
a lens configured to refract the light that has passed through the aperture of the iris diaphragm, and
a slit diaphragm configured for generating slit light from the light refracted by the lens, and
the first optical system is configured to project the slit light generated by the slit diaphragm onto the fundus,
the second optical system includes a focus lens,
the first focus adjusting unit includes a third moving mechanism configured for integrally moving the light source, the iris diaphragm, the lens, and the slit diaphragm in a direction along an optical axis of the first optical system,
the second focus adjusting unit includes a fourth moving mechanism configured for moving the focus lens in a direction along an optical axis of the second optical system, and
the condition determining processor is configured to determine a third movement control condition used for controlling the third moving mechanism as the first focus adjustment condition and determine a fourth movement control condition used for controlling the fourth moving mechanism as the second focus adjustment condition.

9. The ophthalmic apparatus of claim 8, wherein
the third movement control condition includes information indicating a movement direction and a movement distance of the light source, the iris diaphragm, the lens, and the slit diaphragm, and
the fourth movement control condition includes information indicating a movement direction and a movement distance of the focus lens.

10. The ophthalmic apparatus of claim 2, wherein
the processor is configured to control the imaging unit in the slit light projection control to emit the slit light projected onto the fundus from a position spaced a predetermined distance from an optical axis of the imaging unit; and
the condition determining processor is configured to determine the focus adjustment condition based on the position of the slit light image, a focal length of the imaging unit, and the predetermined distance.

11. The ophthalmic apparatus of claim 2, wherein
the imaging unit includes an optical scanner configured to move the projection position of the slit light relative to the fundus by deflecting the slit light guided to the subject's eye,
the processor includes a projection controller configured to perform the slit light projection control, wherein
the projection controller is configured to control the imaging unit to project slit light onto the fundus in a state where a deflection direction of the slit light by the optical scanner is fixed, and
the condition determining processor is configured to determine the focus adjustment condition based on a position of a slit light image corresponding to the slit light projected onto the fundus in the state where the deflection direction is fixed.

12. The ophthalmic apparatus of claim 2, wherein the processor includes a projection controller configured to perform the slit light projection control, wherein
the projection controller is configured to control the imaging unit to output a first slit light and a second slit light whose projection positions on the fundus are different from each other, and
the condition determining processor is configured to determine the focus adjustment condition based on a relative position between a first slit light image corresponding to the first slit light and a second slit light image corresponding to the second slit light.

13. The ophthalmic apparatus of claim 1, wherein
the imaging unit is configured to acquire a time-series image by repeatedly performing a series of imaging using the imaging device while repeating a series of movement of the projection position of the slit light relative to the fundus, and
the processor is further configured to perform
first imaging control for causing the imaging unit to acquire the time-series image;
second imaging control to cause the imaging unit to perform imaging for detecting a focus state of the imaging unit relative to the fundus; and
a focus information generation process to generate focus information indicating the focus state based on an image acquired by the imaging performed under the second imaging control.

14. The ophthalmic apparatus of claim 13, wherein the processor is configured to alternately perform the first imaging control and the second imaging control.

15. The ophthalmic apparatus of claim 13, wherein the processor is further configured to perform display control to output display information based on the focus information to a display device in parallel with the first imaging control, the second imaging control, and the focus information generation process.

16. A method for controlling an ophthalmic apparatus including an imaging unit that performs imaging with an imaging device while moving a projection position of slit light relative to a fundus of a subject's eye, a focus adjusting unit for performing focus adjustment of the imaging unit, a processor, and a memory,
the method comprising causing the processor to perform:
a first control step of controlling the imaging unit to project slit light onto the fundus;
a condition determination step of determining a focus adjustment condition based on an output from the imaging device that has detected return light of the slit light projected by the first control step; and
a second control step of controlling the focus adjusting unit based on the focus adjustment condition determined by the condition determination step.

17. A method for performing imaging with an imaging device while moving a projection position of slit light relative to a fundus of a subject's eye,
the method comprising:
a step of projecting slit light onto the fundus and detecting return light of the slit light projected onto the fundus by the imaging device;
a step of determining a focus adjustment condition based on an output from the imaging device that has detected the return light;
a step of performing focus adjustment based on the focus adjustment condition; and
a step of performing imaging with the imaging device while moving a projection position of slit light relative to the fundus after the focus adjustment has been performed.

18. A program executed by an ophthalmic apparatus including an imaging unit that performs imaging with an imaging device while moving a projection position of slit light relative to a fundus of a subject's eye, a focus adjusting unit for performing focus adjustment of the imaging unit, a processor, and a memory,
the program being configured to cause the processor to perform:
a first control step of controlling the imaging unit to project slit light onto the fundus;
a condition determination step of determining a focus adjustment condition based on an output from the imaging device that has detected return light of the slit light projected by the first control step; and
a second control step of controlling the focus adjusting unit based on the focus adjustment condition determined by the condition determination step.

19. A program for causing a computer including a processor and a memory to execute processing for performing imaging with an imaging device while moving a projection position of slit light relative to a fundus of a subject's eye,
the program being configured to cause the processor to perform:
a step of executing control to project slit light onto the fundus and detect return light of the slit light projected onto the fundus by the imaging device;
a step of determining a focus adjustment condition based on an output from the imaging device that has detected the return light;
a step of performing focus adjustment based on the focus adjustment condition; and
a step of executing control to perform imaging with the imaging device while moving a projection position of slit light relative to the fundus after the focus adjustment has been performed.

20. A computer-readable non-transitory recording medium storing the program of claim 18 or 19.
